# EUROPEAN PATENT SPECIFICATION

(11) **EP 2 928 538 B1**
(45) Date of publication and mention of the grant of the patent: **21.11.2018**
(21) Application number: 13860442.6
(22) Date of filing: 06.12.2013
(51) Int. Cl.: A61F 2/24, A61M 39/22, A61M 25/01

(54) **DEVICES FOR PERCUTANEOUSLY ANCHORING ANNULOPLASTY RINGS**
VORRICHTUNGEN ZUR PERKUTANEN VERANKERUNG VON ANNULOPLASTIERINGEN
DISPOSITIFS D'ANCRAGE PERCUTANÉ D'ANNEAUX D'ANNULOPLASTIE

(30) Priority: 07.12.2012 US 201261734904 P; 13.03.2013 US 201313799642
(43) Date of publication of application: 14.10.2015
(73) Proprietor: Valcare, Inc., Irvine, CA 92618 (US)
(72) Inventor: YELLIN, Nadav, Ramat Gan 5259501 (IL); SHAOLIAN, Samuel, M., Newport Beach, CA 92660 (US); DUMONTELLE, Jeffrey, Irvine, CA 92603 (US)
(74) Representative: Bradley, Adrian
(86) International application number: PCT/US2013/073552
(87) International publication number: WO 2014/089424

(56) References cited:
- US-A1- 2006 241 748
- US-A1- 2007 027 533
- US-A1- 2007 051 377
- US-A1- 2007 051 377
- US-A1- 2007 244 553
- US-A1- 2011 166 649
- US-A1- 2011 301 699
- US-A1- 2012 123 531
- US-A1- 2012 123 531

## Description

### Technical Field

The present disclosure relates to treating and repairing heart valves, and specifically to apparatus, systems, and methods for percutaneous transcatheter repair of heart valves. Disclosed embodiments include adjustable annuloplasty rings that are configured to be delivered through a catheter and percutaneously anchored to a heart valve annulus.

### Background Information

Heart valve defects, such as regurgitation, may be caused by a relaxation of the tissue surrounding a heart valve (e.g., the mitral valve or tricuspid valve). This causes the valve opening to enlarge, which prevents the valve from sealing properly. Such heart conditions are commonly treated by a procedure during which an annuloplasty ring is fixed or secured to the annulus of the valve. Cinching or securing the tissue of the annulus to the annuloplasty ring can restore the valve opening to its approximate original size and operating efficiency.

Typically, annuloplasty rings have been implanted during open heart surgery, so that the annuloplasty ring can be sewn into the valve annulus. Open heart surgery is a highly invasive procedure that requires connecting a heart and lung machine (to pump the patient's blood and breathe for the patient), stopping the patient's heart, and cutting open the thoracic cavity and heart organ. The procedure can expose the patient to a high risk of infection and may result in a long and difficult recovery. The recovery can be particularly difficult for patients in less than optimal health due to the effects of suffering from a heart valve defect such as regurgitation.
US 2006/241748 describes an implantable device for controlling at least one of shape and size of an anatomical structure or lumen.
US 2007/051377 describes a catheter-based annulus reduction device and system for cardiac valve repair.
US 2007/027533 describes a catheter based system for treating mitral valve regurgitation. US 2012/123531 describes apparatus, systems, and method for repairing heart valves through percutaneous transcatheter delivery and fixation of annuloplasty rings to heart valves.

### Summary of the Invention

According to the present invention there is provided an annuloplasty device for transcatheter heart valve repair according to claim 1.

Preferred features of the present invention are recited in the dependent claims.

### Brief Description of the Drawings

FIG. 1A is a top view an internal body member of an annuloplasty ring in an operative geometry.
FIG. 1B is a perspective view of the internal body member shown in FIG. 1A.
FIG. 1C is a side view of the internal body member shown in FIG. 1A in an elongated geometry.
FIG. 2 is a schematic diagram of a cutting pattern used to form the internal body member shown in FIG. 1A.
FIG. 3 is a perspective view of a body member and a device closure of an annuloplasty ring.
FIG. 4A is a perspective view of an anchor deployment system.
FIG. 4B is an enlarged view of the anchor deployment system shown in FIG. 4A.
FIG. 4C is a perspective view of the anchor deployment system shown in FIG. 4A in a partially retracted state.
FIG. 4D is an enlarged view of the anchor deployment system shown in FIG. 4C.
FIG. 4E is a perspective view of the anchor deployment system shown in FIG. 4A in a retracted state.
FIG. 4F is an enlarged view of the anchor deployment system shown in FIG. 4E.
FIG. 5A is a perspective view of a retaining cover used in an anchor deployment system.
FIG. 5B is a perspective view of a body member within the retaining cover shown in FIG. 5A.
FIG. 5C is an enlarged view of the retaining cover of and body member shown in FIG. 5B.
FIG. 5D illustrates the retaining cover shown in FIG. 5C after rotating the body member to deploy an anchor.
FIG. 6 is a perspective view of an annuloplasty ring including an anchor deployment system.
FIG. 7A is a perspective view of annuloplasty ring including another anchor deployment system.
FIG. 7B is a perspective view of a rotation member for use in the anchor deployment system shown in FIG. 7A.
FIG. 7C is a perspective view of another rotation member for use in the anchor deployment system shown in FIG. 7A.
FIG. 7D is an enlarged perspective view of the annuloplasty ring including the anchor deployment system shown in FIG. 7A.
FIG. 7E is a cross-sectional, perspective view of the annuloplasty ring including the anchor deployment system shown in FIG. 7A.
FIG. 8A is top view of an annuloplasty ring in an operative geometry.
FIG. 8B is a schematic diagram of a cutting pattern used to form the annuloplasty ring shown in FIG. 8A.
FIG. 9A is a simplified schematic diagram illustrating a top view of internal anchor ribbons.
FIG. 9B is a simplified schematic diagram illustrating a side view of the internal anchor ribbons shown in FIG. 9A.
FIG. 9C is a simplified schematic diagram illustrating an overlapping side view of the internal anchor ribbons shown in FIG. 9B.
FIG. 9D is a simplified schematic diagram illustrating a top view of a glide ribbon.
FIG. 9E is a simplified schematic diagram illustrating a side view of the glide ribbon shown in FIG. 9D.
FIG. 10A is a simplified schematic diagram illustrating a cross-sectional side view of an annuloplasty ring before anchor deployment.
FIG. 10B is a simplified schematic diagram illustrating a cross-sectional side view of an annuloplasty ring during anchor deployment.
FIG. 10C is a simplified schematic diagram illustrating a cross-sectional side view of an annuloplasty ring after anchor deployment.
FIG. 10D is a perspective view of a portion of an annuloplasty ring after anchor deployment.
FIG. 11A is a perspective view of an annuloplasty ring with separately deployable anchor members with the anchors in an introduction configuration.
FIG. 11B is a perspective view of a separately deployable anchor member.
FIG. 11C is a perspective view of a pair of separately deployable anchor members.
FIG. 11D is a perspective view of an annuloplasty ring with separately deployable anchor members with the anchors in a deployed configuration.
FIG. 12A is a perspective view of an annuloplasty ring with independently deployable anchor members before anchor deployment.
FIG. 12B is a perspective view of an annuloplasty ring with independently deployable anchor members during anchor deployment.
FIG. 12C is a perspective view of an annuloplasty ring with independently deployable anchor members during anchor deployment.
FIG. 12D is a perspective view of an annuloplasty ring with independently deployable anchor members after anchor deployment.
FIG. 13 illustrates various anchor shapes that may be used with the disclosed annuloplasty rings.
FIG. 14 illustrates various anchor designs that may be used with the disclosed annuloplasty rings.
FIG. 15A is a schematic diagram illustrating a trans-septal approach for endovascular delivery of an annuloplasty ring to the mitral valve of a heart.
FIG. 15B is a schematic diagram illustrating an example retrograde approach of an annuloplasty ring to the mitral valve of a heart.
FIG. 15C is a schematic diagram illustrating an example trans-apical approach of an annuloplasty ring to the mitral valve of a heart.
FIG. 16A is a perspective view of an annuloplasty ring in the elongate insertion geometry and partially deployed from the distal end of a delivery catheter in a first deployment stage.
FIG. 16B is a perspective view of an annuloplasty ring in a second stage of partial deployment from the delivery catheter.
FIG. 16C is a perspective view of an annuloplasty ring in a third stage of deployment from the delivery catheter.
FIG. 16D is a perspective view of an annuloplasty ring 1602 in a fourth stage of deployment.
FIG. 17A is a flowchart for a method for repairing a defective heart valve.
FIG. 17B is a flowchart for a method for repairing a defective heart valve.
FIG. 18 is a flowchart for a method for repairing a defective heart valve.

### Detailed Description

While there are flexible rings currently on the market, surgeons generally prefer rigid and semi-rigid rings for valve repair to treat ischemic and functional mitral valve regurgitation. Rigid and semi-rigid rings, unfortunately, do not lend themselves to being delivered into the heart through a catheter. The present disclosure provides systems and methods for repairing heart valves through percutaneous transcatheter delivery and fixation of annuloplasty rings to heart valves. The annuloplasty rings can be configured in both an elongate insertion geometry that can be inserted into a catheter tube and an operable geometry providing a curved and rigid or semi-rigid annular shape.

An annuloplasty ring may be delivered percutaneously to the mitral and/or tricuspid valve annulus of the heart. This may apply, for example, to trans-septal, retrograde, or trans-apical approaches for delivering annuloplasty rings to an annulus of a heart valve. For delivery of rings into the mitral valve, percutaneous delivery may use a retrograde approach from the femoral artery, an antegrade approach via a trans-septal entry, or a trans-apical approach through the base or apex of the heart through the left ventricle to the left atrium. Delivery of rings to the tricuspid valve may include an approach from the inferior or superior vena cava.

Certain annuloplasty rings disclosed herein are small and flexible enough to be percutaneously delivered, but can be put into a rigid or semi-rigid ring shape and then securely anchored into the heart valve annulus without having to open up the chest. Disclosed developments include segmented annuloplasty rings, delivery systems, and methods for anchoring and cinching the annuloplasty ring around the valve annulus.

### Example Rings with Radial, Rotational Anchors

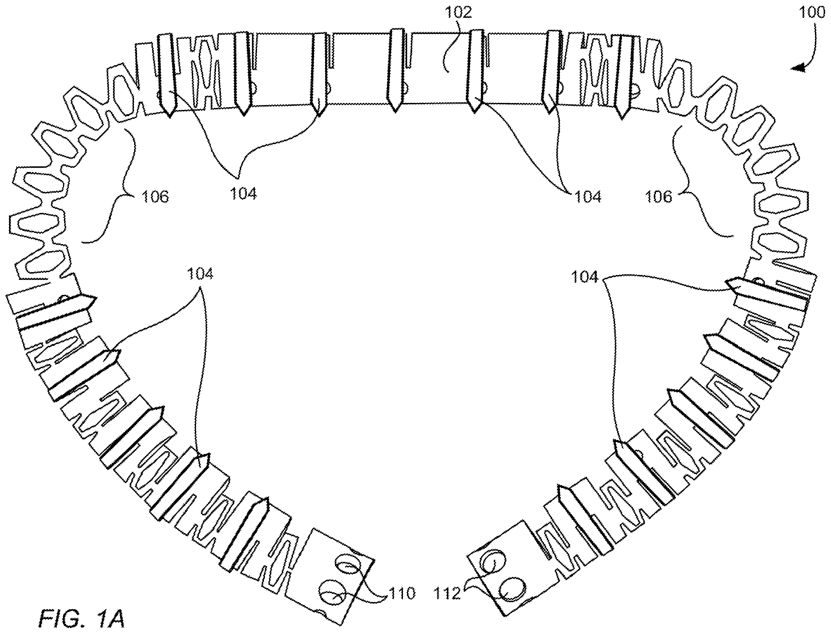

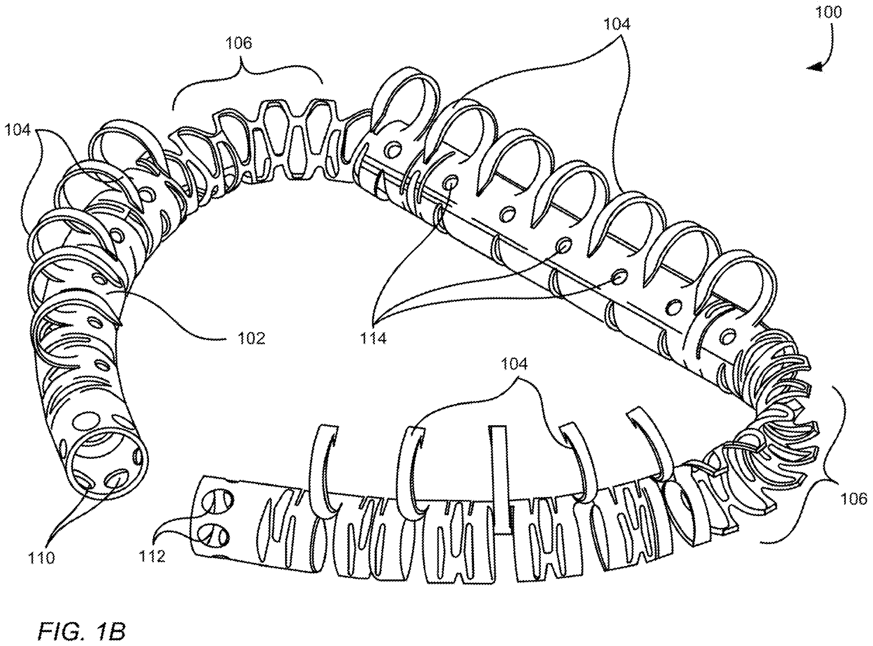

FIGS. 1A and 1B are top and perspective views, respectively, of an internal body member 100 of an annuloplasty ring in an operative geometry according to certain developments. The internal body member 100 includes a hypotube 102 cut to include a plurality of anchors 104 and one or more expansion regions 106. As discussed below, the hypotube 102 may be cut in a pattern (e.g., a crisscross pattern), including in the expansion regions 106, for expansion and compression of the annuloplasty ring to allow transformation from a straight configuration in a delivery system to the operative geometry (e.g., D-shaped or C-shaped) at a delivery site within a heart. The expansion regions 106 may also allow the internal body member 100 to expand when within the heart so that the anchors 104 may be deployed into the heart valve annulus. After anchor deployment in such developments, the internal body member 100 is allowed to contract, which reduces the circumference of the heart valve annulus and reduces regurgitation. The internal body member 100 is configured to enable percutaneous, transcatheter annuloplasty to repair a heart valve. The internal body member 100 may be fastened, percutaneously, to the annulus of the heart valve while in the expanded state and then reduced to the contracted state to decrease an A-P distance of the target valve and thereby improve leaflet coaptation of the target valve and reduce regurgitation through the target valve.

In FIGS. 1A and 1B, as well as in other developments disclosed herein, the internal body member 100 may be arranged in a "D-shape" in the operable geometry (e.g., when implanted around the annulus). The D-shaped body member 100 has a certain geometrical ratio that is in conformance with the anatomical geometry of the annulus of the human mitral or tricuspid valve. For example, in certain developments the ratio of the A-P distance to the commissure-commissure (C-C) distance of the internal body member 100 when implanted (i.e., in the contracted state) is in a range between about 0.60 and about 0.70. In one development, the implanted ratio of the A-P distance to the C-C distance is about 0.62.

In addition to the operable geometry, the internal body member 100 may be placed in an elongate insertion geometry such that the internal body member 100 can be inserted through a catheter into the heart. As discussed in detail below, in certain developments, the hypotube 102 may comprise a shape memory (e.g., Nitinol) hypotube into which a particular pattern is laser cut to form the device features, such as the expansion region 106 and/or anchors 104. The shape memory hypotube 102 is heat set to a "memorized" annular shape (e.g., the D-shaped operable geometry). The shape memory hypotube 102 is superelastic such that applying sufficient stress places the internal body member 100 into the elongate insertion geometry and releasing the stress allows the internal body member 100 to resume the D-shaped operable geometry. Although the illustrated development of an internal body member 100 of FIG. 1A has a D-shaped operable geometry, artisans will recognize from the disclosure herein that other annular-shaped operable geometries may also be used. For example, circular, oval, or C-shaped operable geometries may be used.

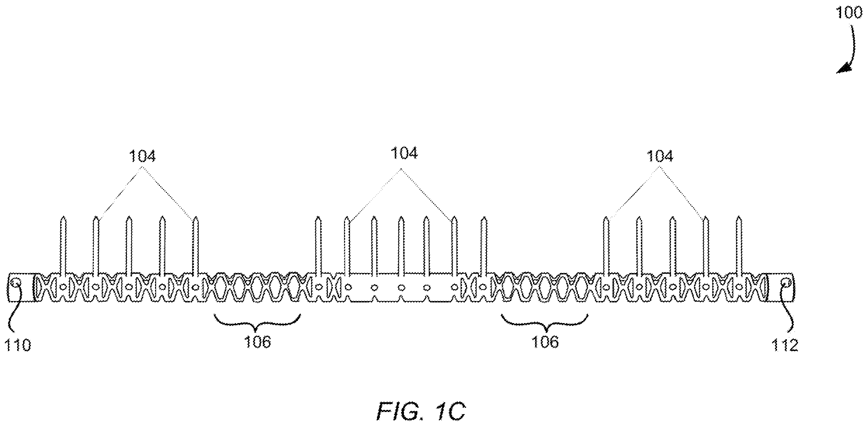

The plurality of anchors 104 are configured to secure the internal body member 100 to the annulus of the heart valve. In certain developments, the anchors 104 are sufficient such that additional suturing of the annuloplasty ring to the valve annulus is not needed. In FIGS. 1A, 1B, and 1C, the anchors 104 are curved in the illustrated deployed configuration. The anchors 104 may be cut from the side of hypotube 102. When the anchors are first cut, they lie in the radial plane of the shape memory hypotube 102 and are concentric with the hypotube 102. The anchors 104 are then heat-set to the curved, deployed configuration. As shown in FIG. 1B, the heat-set anchors curve away from the hypotube 102. The heat-set anchors 104 may curve in the opposite direction from the curvature of the hypotube 102. For example, if newly cut anchors curve to the right, heat-set anchors would curve to the left. Although FIGS. 1A, 1B, and 1C show curved anchors 104, anchors in other developments may include other shapes, such as linear or helical deployed configurations. In certain developments, the anchors 104 include a shape memory material (e.g., Nitinol) that is heat set to a deployed configuration (e.g., linear, helical, or curved configuration shown in FIG. 1B). Artisans will recognize from the disclosure herein that combinations of barb designs and/or deployed configurations may also be used.

In some developments, the anchors 104 are superelastic such that applying sufficient stress places the anchors 104 into an introduction configuration and releasing the stress allows the anchors 104 to resume their respective deployed configurations. In certain developments, the anchors 104 lay in-line with the curvature of the internal body member 100 when in the introduction configuration to facilitate insertion of the internal body member 100 through the catheter. In such developments, the anchors 104 may be selectively deployed at a desired time (e.g., after the internal body member 100 is properly positioned against the annulus of the heart valve). The superelastic property of the anchors 104, combined with the opposite-curvature of the anchors in the introduction and deployed configurations, is used to self-propel the anchors 104 into the annulus of the heart valve. The opposite curvature of the anchors in the introduction and deployed configurations causes the anchors 104 to spring into tissue with extra force. In addition, or in other developments, an operator (e.g., a surgeon) applies a mechanical force to the internal body member 100 to propel the anchors 104 into the annulus of the heart valve.

The hypotube 102 includes one or more through holes 110, 112 at each end to allow one or more pins (not shown) to couple the male and female components of a device closure to respective ends of the hypotube 102. The hypotube 102 may also include a control window that allows one or more lines or sutures to exit the hypotube 102. The lines or sutures are used to snap lock the internal body member 100 into a ring shape and/or to deploy the anchors 104.

In FIG. 1C, the internal body member 100 is shown in the elongate insertion geometry. It should be noted, however, that the anchors 104 in FIG. 1C are shown in the extended (deployed) position, and not in a configuration suitable for insertion through a catheter. As discussed below, the internal body member 100 is formed from a straight (elongate) hypotube into which features, such as anchors 104, may be formed.

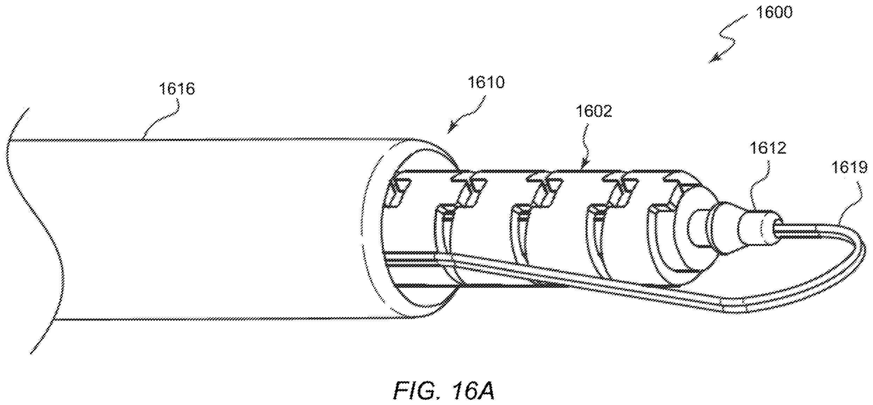

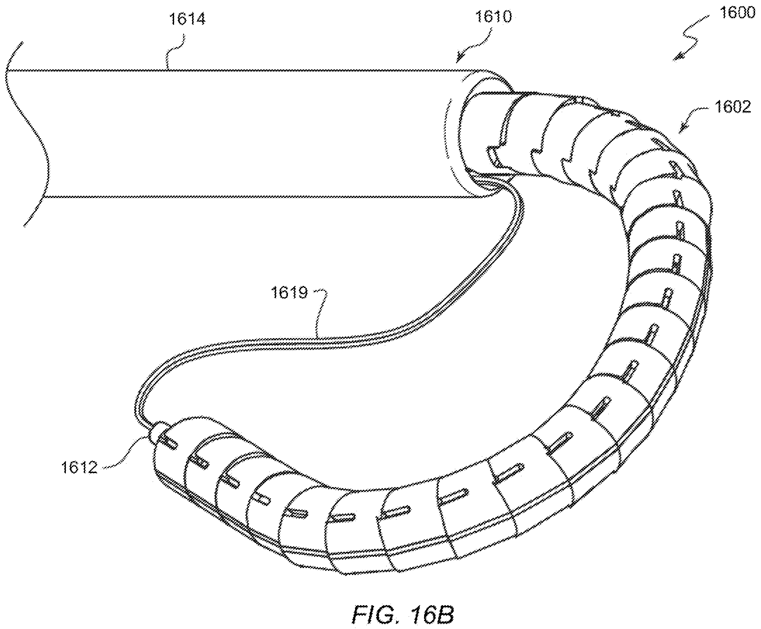

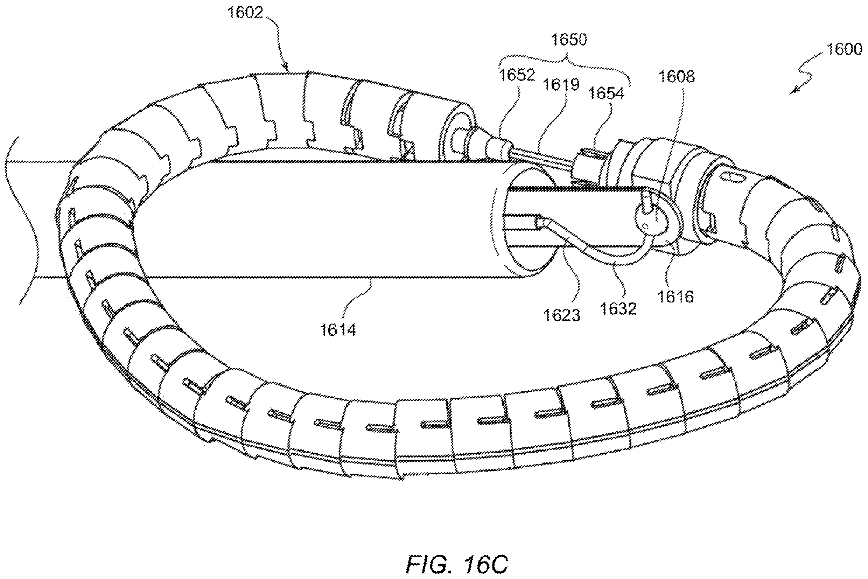

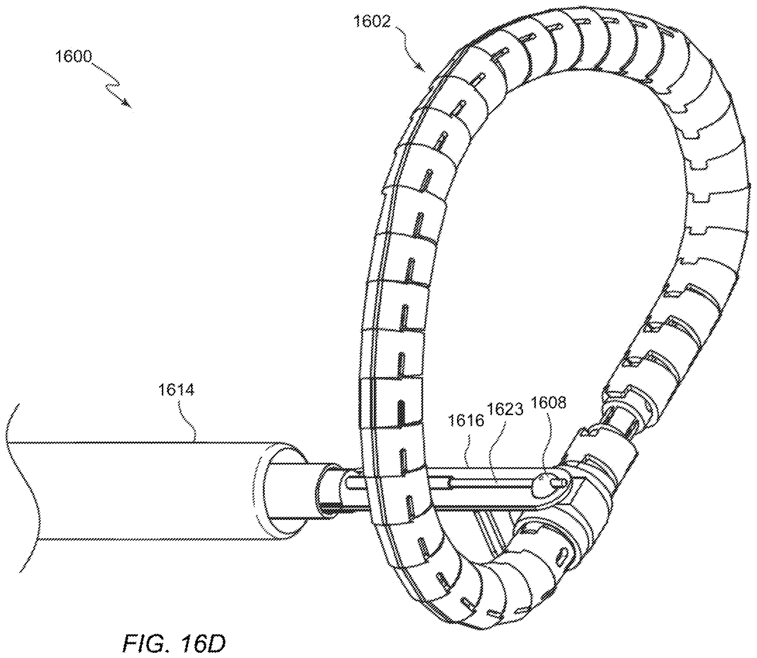

In addition to the operable geometry shown in FIGS. 1A and 1B the internal body member 100 is capable of making the transition from an elongate insertion geometry (FIG. 1C) to the annular operable geometry (FIGS. 1A and 1B). The elongate insertion geometry allows the internal body member 100 to be inserted into and passed through a catheter for percutaneous passage of the annuloplasty ring 600 into the heart of a patient. A transition from an elongate insertion geometry to an annular operable geometry is illustrated in FIGS. 16A, 16B, 16C, and 16D, and discussed below with reference to the same.

Although not shown in FIGS. 1A, 1B, and 1C, certain ring developments may include a selectively adjustable member for changing the size and/or shape of the internal body member 100 postoperatively to compensate for changes in the size of the heart and/or the treated heart valve. Examples of a selectively adjustable member are provided in U.S. Patent Application No. 13/198,582, filed August 4, 2011, and titled PERCUTANEOUS TRANSCATHETER REPAIR OF HEART VALVES.

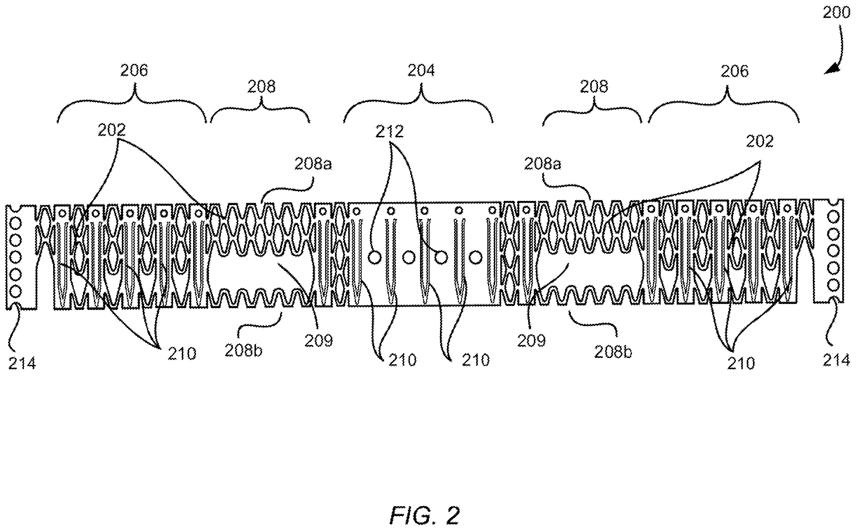

FIG. 2 is a schematic diagram of a cutting pattern 200 used for laser processing hypotubes, such as the hypotube 102 shown in FIGS. 1A, 1B, and 1C. The cutting pattern 200 corresponds to the entire hypotube 102 as if the hypotube 102 were cut along a longitudinal axis and unrolled. Thus, for example, each expansion region 106 shown in FIG. 1C is shown in FIG. 2 as being split between a first half of the expansion region 208a and a second half of the expansion region 208b.

The cutting pattern 200 defines the configuration of a hypotube (e.g., hypotube 102) and defines how regions of the hypotube (e.g., expansion regions 106) interact with adjacent regions as the hypotube transitions from an elongate insertion geometry (see e.g., FIG. 1C) to an annular operable geometry (see e.g., FIGS. 1A and 1B). As shown in FIG. 2, the cutting pattern 200 in this example development includes a "crisscross" pattern 202. The crisscross pattern 202 allows for increased expansion and/or contraction in regions of the hypotube containing the crisscross pattern 202. One or more rigid regions 204, semi-rigid regions 206, and expansion regions 208 may be defined using the crisscross pattern 202. The rigid region 204 does not include the crisscross pattern 202. The semi-rigid region 206 includes an intermittent crisscross pattern 202. The crisscross pattern 202 is throughout the expansion region 208. The expansion region 208 may also include large gaps or cutout regions 209 to allow increased flexibility or bending of the internal body member (e.g., at the corners of the D-shape as shown in FIG. 1B at the expansion regions 106). The cutting pattern 200 also includes anchor cutouts 210, control window cutouts 212, and through hole cutouts 214. The anchor cutouts 210 define the length, width, and location of annuloplasty ring anchors, such as anchors 104. Similarly, the control window cutouts 212 and through hole cutouts 214 define the size, geometry, and location of control windows and through holes in the hypotube. The cutting pattern 200 defines the annular operable geometry of a hypotube, allows the hypotube to easily transition from the elongate insertion geometry to the annular operable geometry, and allows for adjustment in the anterior-posterior (A-P) dimension to ensure that the annulus is sufficiently reduced.

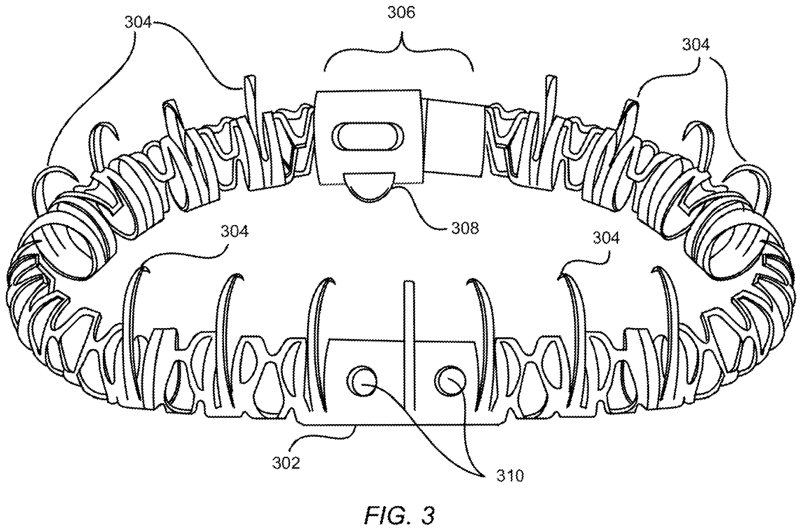

FIG. 3 is a perspective view of a body member 302 and a device closure 306 of an annuloplasty ring for percutaneous, trans-catheter annuloplasty repair according to certain developments. The illustrated body member 302 may be similar to the internal body member 100 discussed above in reference to FIGS. 1A, 1B, and 1C..

The body member 302 is laser cut into a desired shape. A cutting pattern, such as cutting patter 200 shown in FIG. 2, may be used to define regions of the body member 302 where the body member 302 may be flexible or rigid. In certain developments, anchors 304 are cut from the body member 302. In other development, the anchors 304 may be attached to body member 302 by welding, adhesive, or other suitable fastening means. The anchors 304 are configured to attach the annuloplasty ring to the heart valve annulus.

The device closure 306 is used to secure two open ends of the annuloplasty ring to form a closed ring of the operable geometry. In certain developments, a ring closure lock includes a female snap and a male snap. Examples of ring closure locks are provided in U.S. Patent Application No. 13/779,478.

A pivot 308 is attached to body member 302 and/or the device closure 306 and is used to rotate the annuloplasty ring after deployment via catheter within the heart to align the plane of the annuloplasty ring (in the annular operable geometry) with the plane of the heart valve. The pivot 308 may be manually coupled (e.g., using a pin, etc.), welded, or bonded using an adhesive. Upon exiting the catheter, the annuloplasty ring may be rotated via the pivot 308 to allow the annuloplasty ring to be properly positioned against the heart valve annulus. In some developments, the pivot 308 is used to rotate the annuloplasty ring such that the anchors 304 are propelled into the surrounding annular tissue.

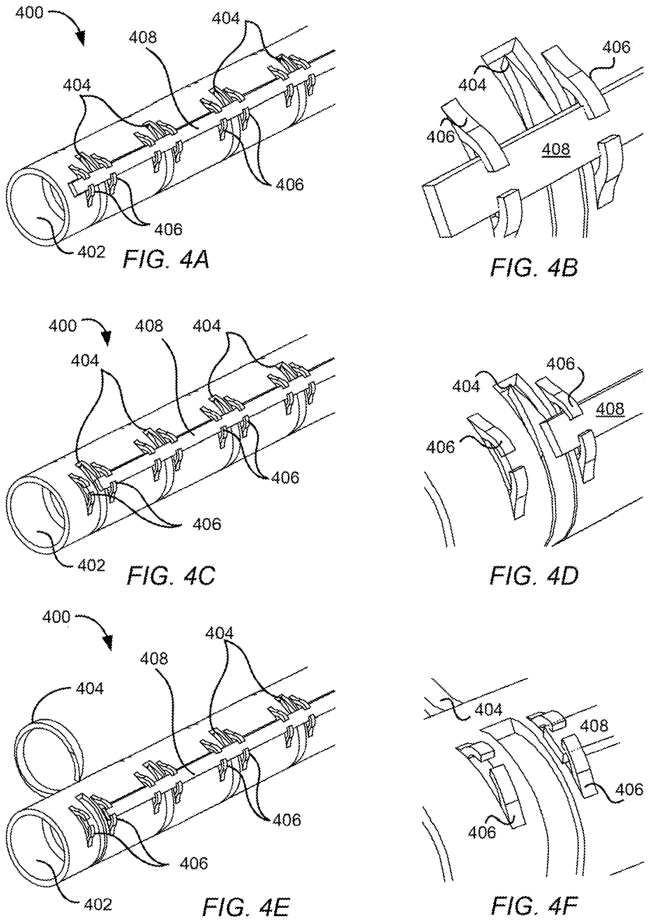

FIGS. 4A, 4B, 4C, 4D, 4E, and 4F are perspective views of an anchor deployment system 400 for deploying anchors of an annuloplasty ring into a heart valve annulus. The system 400 includes a body member 402 with a plurality of anchors 404, a plurality of retainer features 406, and a retaining ribbon 408. The body member 402 also includes the body members 100, 300 discussed above in reference to FIGS. 1A, 1B, and 1C and FIG. 3. The anchors 404 are heat treated to extend away from the body member 402 so as to anchor the body member 402 into heart valve annulus tissue. In some embodiments, the anchors 404 curve away from the body member 402. The anchors 404 may curve in the opposite direction from the body member 402. For example, if newly cut anchors curve to the right, heat-set anchors would curve to the left, similar to the anchors 104 shown in FIG. 1B.

Referring again to FIGS. 4A, 4B, 4C, 4D, 4E, and 4F, certain annuloplasty ring embodiments include the retaining ribbon 408 disposed about an outer circumference of the body member 402, or selected portions thereof. The retaining ribbon 408 prevents the anchors 404 from deploying until the annuloplasty ring is properly positioned against the heart valve annulus. The retainer features 406 are disposed in pairs on either side of each anchor 404 and are configured to hold the retaining ribbon 408 against the surface body member 402. The plurality of anchors 404 have superelastic properties allowing them to spring back into their heat-set shape as described above. Additionally, the opposite-curvature of the anchors causes the anchors 404 to spring into tissue with extra force. Prior to deployment, stress is applied to anchors 404 causing them to lie flush with the body member 402. The retaining ribbon 408 is inserted between the anchors 404 and the retainer features 406 thereby holding the anchors 404 flush with the body member 402. In some embodiments, when the body member 402 is configured in the operable geometry and positioned around the annulus, the retaining ribbon 408 is retracted so that the anchors 404 deploy. When the retaining ribbon 408 retracts past an anchor 404, the stress on the anchor 404 is released causing the anchor 404 to self-deploy into the annulus tissue. In other embodiments, the anchors 404 are deployed before the body member 402 is placed in the operable geometry.

FIGS. 4A and 4B are views of an anchor deployment system 400 wherein the retaining ribbon 408 is disposed between the anchors 404 and the retainer features 406. FIG. 4B is an enlarged view of the anchor deployment system of FIG. 4A showing how the retaining ribbon 408 is disposed between an anchor 404 and a pair of retainer features 406 thereby preventing the anchor 404 from deploying.

FIGS. 4C and 4D are views of the anchor deployment system 400 when the retaining ribbon 408 is partially retracted past an anchor 404. In some embodiments, the strength and/or rigidity of the retaining ribbon 408 is sufficient to prevent the anchor 404 from deploying until the retaining ribbon 408 has retracted across the entire anchor 404. In other embodiments, the strength and/or rigidity of the retaining ribbon 408 is sufficient to prevent the anchor 404 from deploying until the retaining ribbon 408 has retracted halfway across the anchor 404. In yet other embodiments, the strength and/or rigidity of the retaining ribbon 408 is only sufficient to prevent the anchor 404 from deploying until the retaining ribbon 408 has retracted past the first retainer feature 406 of the pair of retainer features 406 surrounding the anchor 404. FIG. 4D is an enlarged view of the anchor deployment system of FIG. 4C showing how the retaining ribbon is partially retracted across an anchor 404.

FIGS. 4E and 4F are views of the anchor deployment system 400 when the retaining ribbon 408 is fully retracted past an anchor 404. When the force applied to an anchor 404 by the retaining ribbon 408 becomes insufficient, the anchor 404 self-propels into the annulus tissue thereby anchoring the annuloplasty ring into the heart valve annulus. FIG. 4F is an enlarged view of the anchor deployment system of FIG. 4E showing the retaining ribbon fully retracted past an anchor 404.

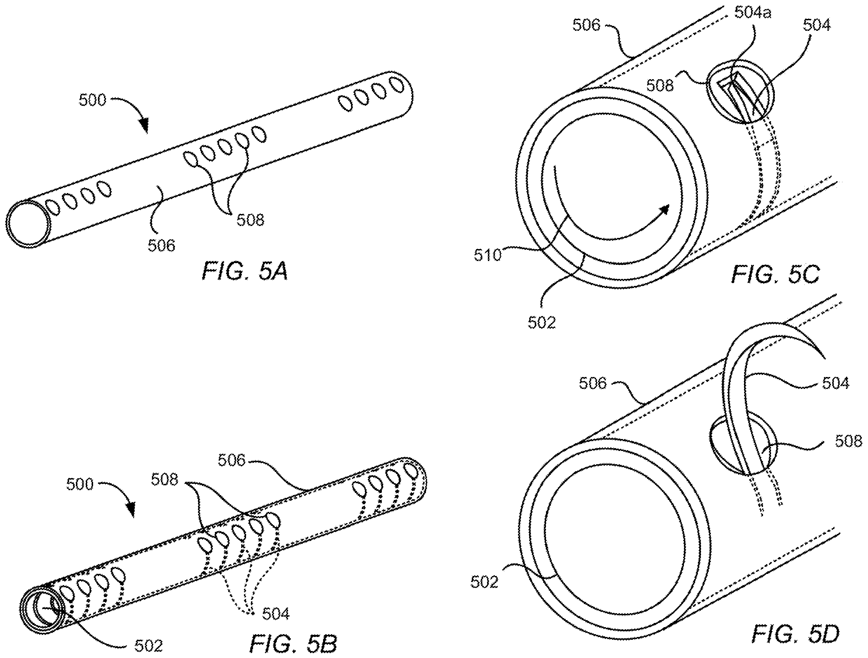

FIGS. 5A, 5B, 5C, and 5D are perspective views of an anchor deployment system 500 for deploying anchors of an annuloplasty ring into a heart valve annulus according to certain developments. The system 500 includes a body member 502 with a plurality of anchors 504, and an outer tube or retaining cover 506. In some developments, the retaining cover 506 includes a plurality of deployment windows 508 through which the plurality of anchors 504 are deployed, each deployment window 508 corresponding to an anchor 504. In yet other developments, the plurality of anchors 504 are configured to puncture through the retaining cover 506 during deployment. The body member 502 may be similar to the body member 100, 300, and 402 discussed above in reference to FIGS. 1A, IB, and 1C, 3, and 4A, 4B, 4C, 4D, 4E, and 4F. The anchors 504 have been heat treated to extend away from the annuloplasty ring 502 so as to anchor the annuloplasty ring 502 into heart valve annulus tissue.

Certain annuloplasty ring developments include an outer tube or retaining cover 506 disposed about the entire circumference of the annuloplasty ring 502, or selected portions thereof. For example, in certain developments, the retaining cover 506 is disposed so as to enclose the anchors 504 and the expansion region(s), while leaving uncovered at least portions of a closure mechanism (to permit snapping the ends of the annuloplasty ring together). The retaining cover 506 prevents the anchors 504 from deploying until the annuloplasty ring is properly positioned against the heart valve annulus. The plurality of anchors 504 have superelastic properties allowing them to spring back into their heat-set shape as described above. The superelastic property of the anchors 504, combined with the opposite-curvature of the anchors, is used to self-propel the anchors 504 into the annulus of the heart valve. The opposite curvature of the anchors 504 causes them to spring into tissue with extra force. Prior to deployment, stress is applied to anchors 504 causing them to lie flush with the body member 502. The body member 502, including anchors 504, is inserted into the retaining cover 506 thereby holding the anchors 504 flush with the body member 502 and preventing their deployment. In some developments, when the annuloplasty ring is configured in the operable geometry and positioned around the annulus, the body member 502 is rotated within the retaining cover 506. When the anchors 504 are rotated to the deployment windows 508, the stress on the anchors 504 is released, allowing the anchors 504 to self-deploy into the annulus tissue. In other developments, the anchors 504 are deployed before the annuloplasty ring 502 is placed in the operable geometry.

FIG. 5A is a perspective view of the retaining cover 506 including the plurality of deployment windows 508. The retaining cover 506 may include a biocompatible material such as Dacron®, woven velour, polyurethane, polytetrafluoroethylene (PTFE), heparin- coated fabric, or the like. In other developments, the retaining cover 506 includes a biological material such as bovine or equine pericardium, homograft, patient graft, or cell-seeded tissue.

FIG. 5B is a perspective view of the body member 502 after being inserted into the retaining cover 506. In FIG. 5B, the body member 502 is in the elongated configuration. After being deployed into the heart through a catheter, the body member 502 is placed into an annular operable geometry. The retaining cover 506 is flexible and continues to surround the annuloplasty ring 502 while in the operable geometry.

FIG. 5C is an enlarged perspective view of the anchor deployment system 500. As seen in FIG. 5C, the tip 504a of the anchor 504 is at the deployment window 508, but the body member 502 may be rotated further before the anchor 504 deploys into the annular tissue. As the body member 502 rotates in the direction of arrow 510, the elastic force of the anchor 504 causes the tip 504A to protrude from the deployment window 508. Further rotation in the direction of the arrow 510 causes more of the anchor 504 to protrude beyond the retaining cover 506 as the anchor deploys.

FIG. 5D is an enlarged perspective view of the anchor deployment system 500. As seen in FIG. 5D, the continued rotation of the body member 502 causes the anchor 504 to deploy. Referring back to FIGS. 5A and 5B, in the illustrated development, the deployment windows 508 are aligned with each other causing all the anchors 504 to simultaneously deploy as the body member 502 rotates within the retaining cover 506. In other developments, the deployment windows 508 may be disposed on the retaining cover 506 in an unaligned manner so that the anchors 504 deploy in a desired sequence. Where simultaneous deployment is not desired, the deployment windows 508 may have oval, oblong, or rectangular shapes so that continued rotation will not retract deployed anchors or displace the annuloplasty ring 502 from its position around the heart valve annulus.

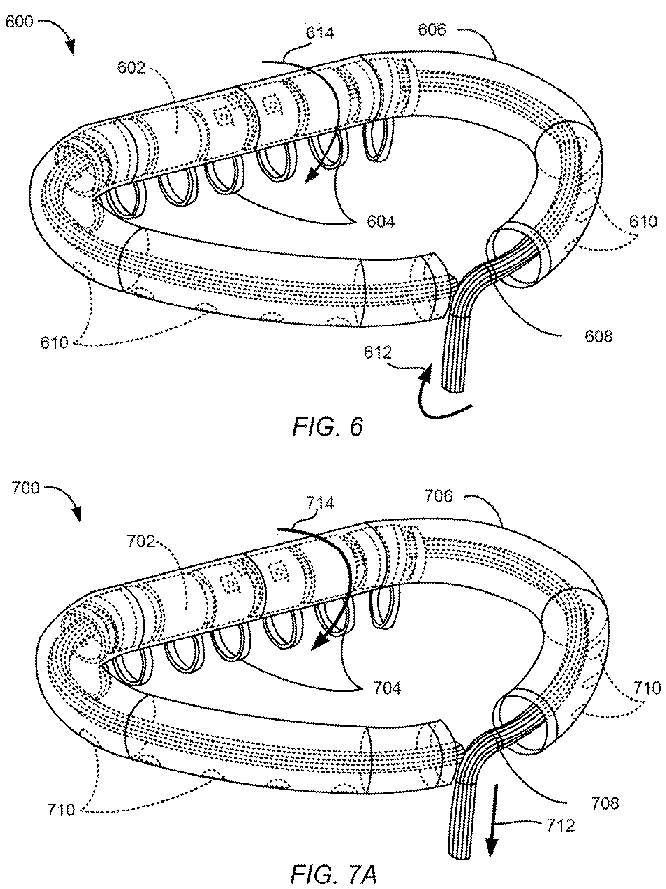

FIG. 6 is a perspective view of an annuloplasty ring 600 including an anchor deployment system according to certain developments. In some developments, the anchor system shown in FIG. 6 may be combined with the anchor deployment system 500 shown in FIG. 5. The annuloplasty ring 600 includes a shape memory body member 602, a plurality of anchors 604, an outer tube 606, and a deployment wire 608. The deployment wire 608 is attached to the body member 602 and is used to selectively deploy the anchors 604. The plurality of anchors 604 are attached to the shape memory body member 602. In some developments, the anchors 604 are welded to the shape memory body member 602. In other developments, the anchors 604 are laser cut from the side of the shape memory body member 602 and then heat-set to a curved, deployed configuration, similar to the anchors 104 discussed above in reference to FIGS. 1A, 1B, and 1C. In some developments, the anchors 604 are curved when in the deployed configuration. The anchors 604 may curve away from the body member 602. In some developments, the anchors 604 curve in the opposite direction from the body member 602. The anchors 604 in other developments may include other shapes, such as linear or helical deployed configurations. In certain developments, the anchors 604 include a shape memory material (e.g., Nitinol) that is heat set to a deployed configuration (e.g., linear, helical, or curved). Artisans will recognize from the disclosure herein that combinations of barb designs and/or deployed configurations may also be used.

In some developments, the anchors 604 are superelastic such that applying sufficient stress places the anchors 604 into an introduction configuration and releasing the stress allows the anchors 604 to resume their respective deployed configurations. In certain developments, the anchors 604 lay in-line with the body member 602 when in the introduction configuration to facilitate insertion of the annuloplasty ring 600 through a catheter. The body member 602, including the anchors 604, is inserted into the outer tube 606 thereby holding the anchors 604 flush with the body member 602 and preventing their deployment. In some developments, the body member 602 runs the full length of the outer tube 606. In other developments, the body member 602 is segmented into multiple segments. The outer tube 606 prevents the anchors 604 from deploying until properly positioned against the heart valve annulus. In such developments, the anchors 604 may be selectively deployed at a desired time (e.g., after the body member 602 is properly positioned against the annulus of the heart valve). The superelastic property of the anchors 604, combined with the opposite curvature of the anchors, causes the anchors 604 to spring into tissue with extra force.

When the annuloplasty ring 600 is configured in an operable geometry and positioned around the annulus, the body member 602 is rotated within the outer tube 606 via the deployment wire 608, causing the anchors 604 to deploy. The deployment wire 608 passes inside the outer tube 606 and attaches to the body member 602. The deployment wire 608 is a torque wire capable of applying torque to the body member 602. To deploy the anchors, the deployment wire 608 is rotated in the direction of arrow 612, which results in the body member 602 rotating in the direction of arrow 614. As the body member 602 rotates, the anchors 604 deploy through the outer tube 606.

In some developments, the outer tube 606 includes a plurality of anchor deployment windows 610 that allow the anchors 604 to pass through the outer tube 606. When the anchors 604 are rotated within the outer tube 606 to the deployment windows 610, the stress on the anchors 604 is released allowing the anchors 604 to deploy. The superelastic property of the anchors 604, combined with the opposite curvature of the anchors, causes the anchors 604 to vigorously spring out of the deployment windows 610.

The outer tube 606 may include a biocompatible material such as Dacron®, woven velour, polyurethane, polytetrafluoroethylene (PTFE), heparin-coated fabric, or the like. In other developments, the outer tube 606 includes a biological material such as bovine or equine pericardium, homograft, patient graft, or cell-seeded tissue.

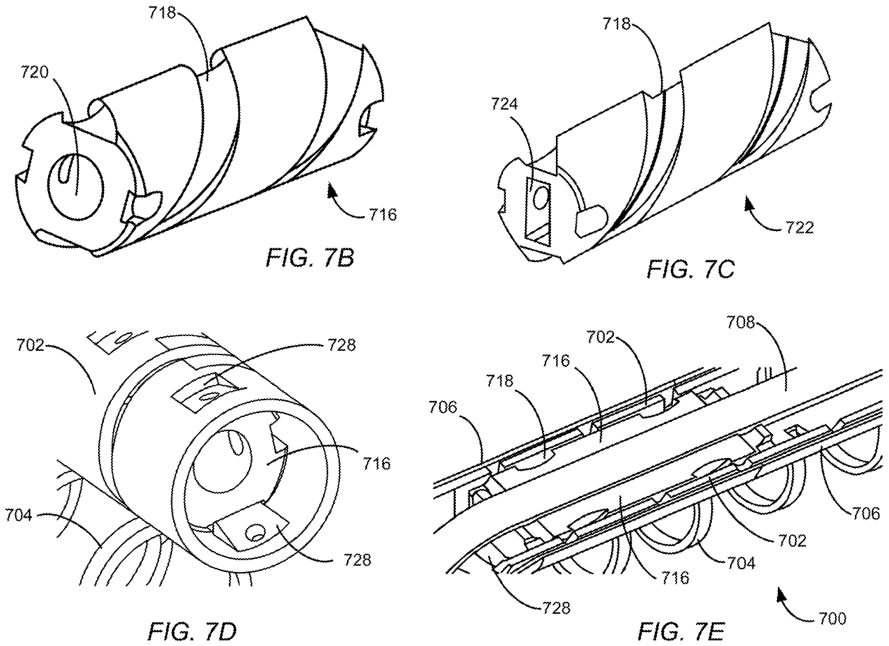

FIG. 7A is a perspective view of an annuloplasty ring 700 including another anchor deployment system according to the present disclosure. In some developments, the anchor system shown in FIG. 7 may be combined with the anchor deployment system 500 shown in FIG. 5. The anchor deployment system is used to deploy anchors of an annuloplasty ring 700, such as those described in the present disclosure. The annuloplasty ring 700 includes a shape memory body member 702, a plurality of anchors 704, an outer tube 706, and a deployment wire 708. The deployment wire 708 is attached to the body member 702 and is used to selectively deploy the anchors 704. The plurality of anchors 704 are attached to the shape memory body member 702. In some developments, the anchors 704 are welded to the shape memory body member 702. In other developments, the anchors 704 are laser cut from the side of the shape memory body member 702 and then heat-set to a curved, deployed configuration, similar to the anchors 104 discussed above in reference to FIGS. 1A, 1B, and 1C. In some developments, the anchors 704 are curved when in the deployed configuration. The anchors 704 may curve away from the body member 702. In some developments, the anchors 704 curve in the opposite direction from the body member 702. The anchors 704 in other developments may include other shapes, such as linear or helical deployed configurations. In certain developments, the anchors 704 include a shape memory material (e.g., Nitinol) that is heat set to a deployed configuration (e.g., linear, helical, or curved). Artisans will recognize from the disclosure herein that combinations of barb designs and/or deployed configurations may also be used.

In some developments, the anchors 704 are superelastic such that applying sufficient stress places the anchors 704 into an introduction configuration and releasing the stress allows the anchors 704 to resume their respective deployed configurations. In certain developments, the anchors 704 lay in-line with the body member 702 when in the introduction configuration to facilitate insertion of the body member 702 through the catheter. The body member 702, including anchors 704, is inserted into the outer tube 706 thereby holding the anchors 704 flush with the body member 702 and preventing their deployment. In some developments, the body member 702 runs the full length of the outer tube 706. In other developments, the body member 702 is segmented into multiple segments. The outer tube 706 prevents the anchors 704 from deploying until properly positioned against the heart valve annulus. In such developments, the anchors 704 may be selectively deployed at a desired time (e.g., after the annuloplasty ring is properly positioned against the annulus of the heart valve). The superelastic property of the anchors 704, combined with the opposite curvature of the anchors, causes the anchors 704 to spring into tissue with extra force.

When the annuloplasty ring 700 is configured in an operable geometry and positioned around the annulus, the body member 702 is rotated within the outer tube 706 via the deployment wire 708, causing the anchors 704 to deploy. The deployment wire 708 passes inside the outer tube 706 and is coupled to a rotation member (not shown). The rotation member transforms linear movement of the deployment wire 708 into rotational movement of the body member 702. To deploy the anchors 704, the deployment wire 708 is pulled in the direction of arrow 712, which results in the body member 702 rotating in the direction of arrow 714. As the body member 702 rotates, the anchors 704 deploy through the outer tube 706.

In some developments, the outer tube 706 includes a plurality of anchor deployment windows 710 that allow the anchors 704 to pass through the outer tube 706. When the anchors 704 are rotated within the outer tube 706 to the deployment windows 710, the stress on the anchors 704 is released allowing the anchors 704 to deploy. The superelastic property of the anchors 704, combined with the opposite curvature of the anchors, causes the anchors 704 to vigorously spring out of the deployment windows 710.

The outer tube 706 may include a biocompatible material such as Dacron®, woven velour, polyurethane, polytetrafluoroethylene (PTFE), heparin-coated fabric, or the like. In other developments, the outer tube 706 includes a biological material such as bovine or equine pericardium, homograft, patient graft, or cell-seeded tissue.

FIG. 7B is a perspective view of a rotation member 716 for use with the anchor deployment system 700 shown in FIG. 7A according to certain developments. The rotation member 716 includes one or more threaded grooves 718 configured to apply torque to the body member 702. The rotation member 716 further includes a hole 720 through which a round wire (such as deployment wire 708) may pass.

FIG. 7C is a perspective view of a rotation member 722 for use with the anchor deployment system 700 shown in FIG. 7A according to certain developments. The rotation member 722 includes one or more threaded grooves 718 configured to apply torque to the body member 702. The rotation member 722 further includes a hole 724 through which a flat wire or ribbon (such as deployment wire 708) may pass.

FIG. 7D is an enlarged perspective view of the annuloplasty ring including the anchor deployment system shown in FIG. 7A. In some developments, the body member 702 includes one or more inner tabs 728 configured to engage the threaded grooves 718 of the rotation member 716 shown in FIG. 7B (or the rotation member 722 shown in FIG. 7C). As the deployment wire 708 moves linearly (e.g., is pushed or pulled by a user), the rotation member 716 applies torque to the body member 702 via the groove 718 moving along the inner tabs 728. The rotation of body member 702 causes anchors 704 to deploy.

FIG. 7E is a cross-sectional, perspective view of the annuloplasty ring including the anchor deployment system shown in FIG. 7A. As shown, the deployment wire 708 passed through rotation member 716. Rotation member 716 is located inside body member 702 and the threaded grooves 718 engage the inner tabs 728 to cause body member 702 to rotate within outer tube 706. When the rotation of body member 702 causes the anchors 704 to reach the deployment windows 710, the anchors 704 deploy through the outer tube 706.

### Example Ring Developments with Separately Deployed Anchors

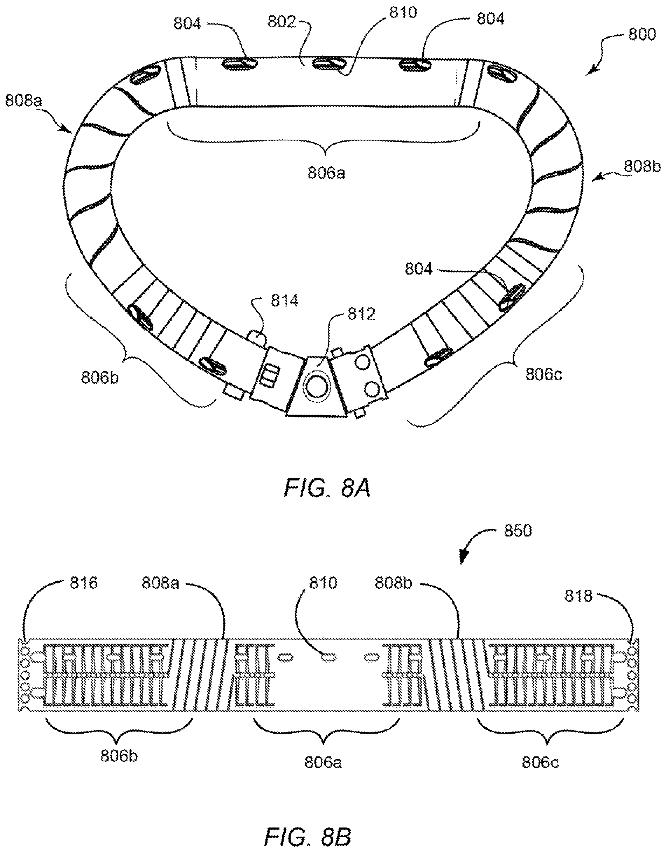

FIG. 8A is top view of an annuloplasty ring 800 in an operative geometry according to certain developments. The annuloplasty ring 800 illustrated in FIG. 8A is in an annular (D-shaped) operable geometry in a contracted state. The annuloplasty ring 800 is configured to enable percutaneous, transcatheter annuloplasty repair of a heart valve. The annuloplasty ring 800 may be fastened, percutaneously, to the annulus of the heart valve while in the expanded state and then reduced to the contracted state to decrease an A-P distance of the target valve and thereby improve leaflet coaptation of the target valve and reduce regurgitation through the target valve.

The annuloplasty ring 800 includes a body member 802 having a plurality of anchors 804, a plurality of anchor regions 806a, 806b, 806c (collectively 806), a plurality of expansion regions 808a, 808b (collectively 808), a plurality of anchor windows 810, a ring closure lock 812, and a pivot 814. In some developments, the anchors 804 may be separately deployed through the anchor windows 810. In FIG. 8A the body member 802, including the plurality of regions 806, 808, is arranged in a "D-shape" in the operable geometry. The D- shaped annuloplasty ring 800 has a certain geometrical ratio that is in conformance (or approximate conformance) with the anatomical geometry of the human mitral valve annulus. For example, in certain developments the ratio of the A-P distance to the commissure- commissure (C-C) distance of the annuloplasty ring 800 when implanted (i.e., in the contracted state) is in a range between about 0.60 and about 0.70. In one development, the implanted ratio of the A-P distance to the C-C distance is about 0.62.

Although the illustrated development of an annuloplasty ring 800 of FIG. 8A is a D-shaped operable geometry, artisans will recognize from the disclosure herein that other annular-shaped operable geometries may also be used. For example, circular, oval, or C- shaped operable geometries may be used.

The body member 802 may be cut from, for example, a tube to form the plurality of regions 806, 808. The cuts may define a shape and/or characteristics of the body member 802. For example, the laser cuts may define the anchor regions 806, expansion regions 808, and/or the anchor windows 810. The laser cuts may also define how the plurality of regions 806, 808 interacts.

In certain developments, the body member 802 may include a shape memory (e.g., Nitinol) hypotube into which a plurality of cuts and/or segments may be laser cut to define a size, shape, and/or characteristics of the plurality of regions 806, 808. The shape memory body member 802 may be heat set to a "memorized" annular shape (e.g., the D-shaped operable geometry). The shape memory body member 802 may be superelastic such that applying sufficient stress may place the annuloplasty ring 800 into the elongate insertion geometry and releasing the stress allows the annuloplasty ring 800 to resume the D-shaped operable geometry.

In addition to the operable geometry shown in FIG. 8A the annuloplasty ring 800 is transitionable from an elongate insertion geometry to the annular operable geometry shown in FIG. 8A. The elongate insertion geometry allows the annuloplasty ring 800 to be inserted into and passed through a catheter for percutaneous passage of the annuloplasty ring 800 into the heart of a patient. A transition from an elongate insertion geometry to an annular operable geometry is illustrated in FIGS. 16A, 16B, 16C, and 16D, and discussed below with reference to the same.

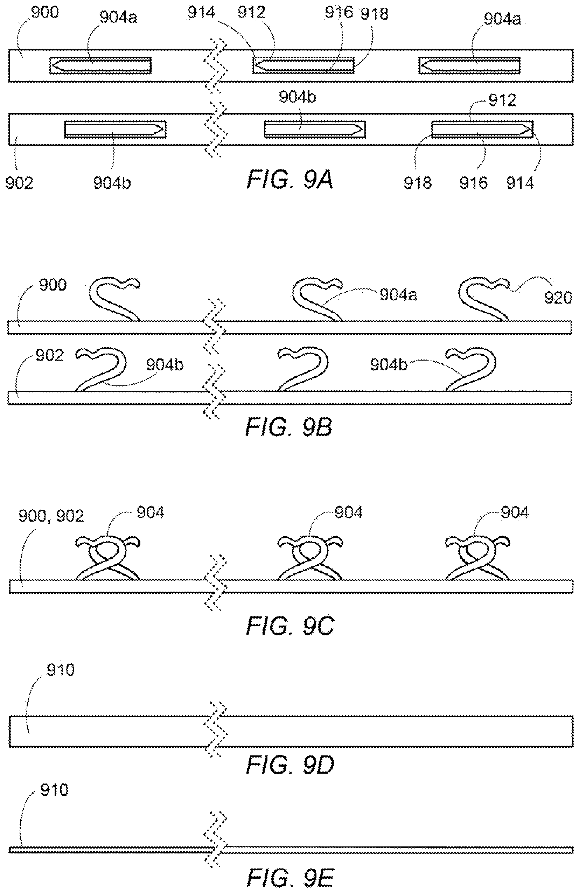

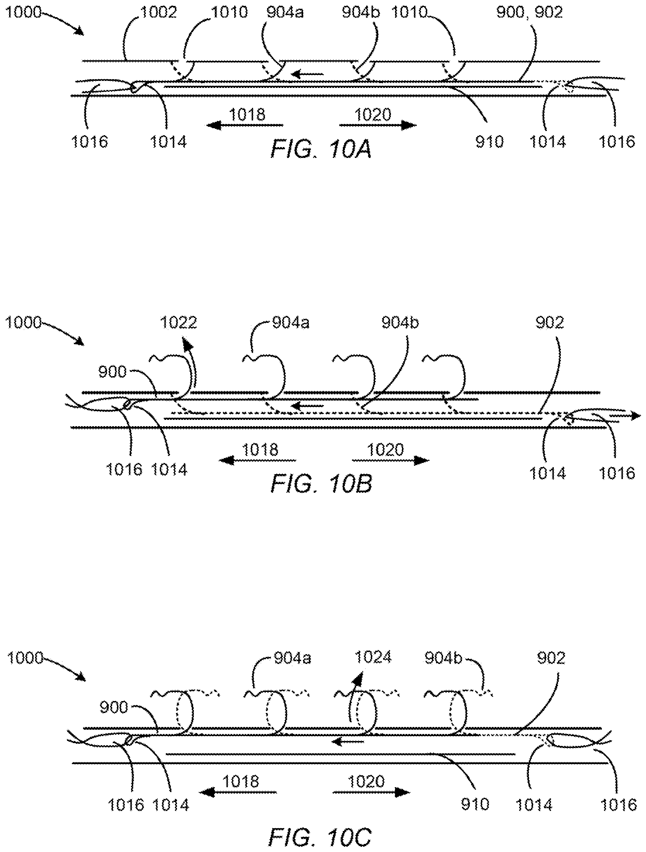

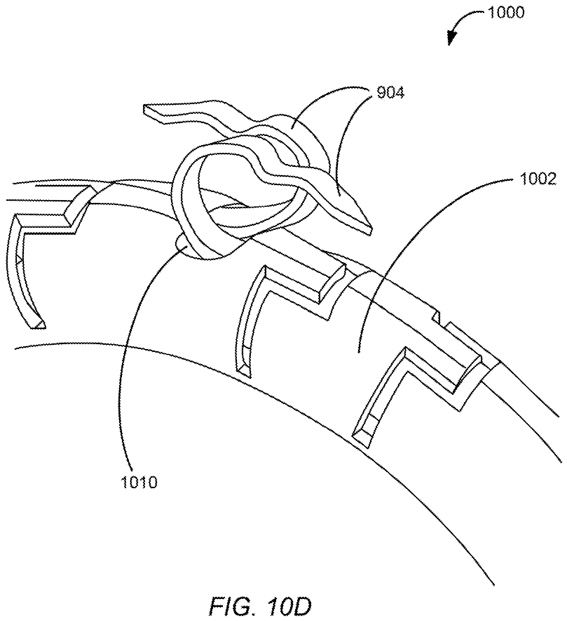

The anchors 804 are configured to secure the annuloplasty ring 800 to the annulus of the heart valve. In certain developments, the anchors 804 are sufficient such that additional suturing of the annuloplasty ring 800 to the valve annulus is not needed. In FIG. 8A the anchors 804 are within the body member 802 in an introduction configuration. The anchors 804 may be later deployed, as discussed below with reference to FIGS. 9A, 9B, 9C, 9D, and 9E and FIGS. 10A, 10B, 10C, and 10D. The anchors 804 may be selected from a plurality of shapes, such as curved, linear, or helical deployed configurations. In certain developments, the anchors 804 include a shape memory material (e.g., Nitinol) that is heat set to a deployed configuration (e.g., curved configuration, linear configuration, or helical configuration). Artisans will recognize from the disclosure herein that combinations of different deployed anchor configurations may also be used.

In some developments, the anchors 804 are in the same plane as the annuloplasty ring. In other developments, the anchors 804 deploy at an angle to the plane of the annuloplasty ring, such as 10°, 45°, or even 90°.

In some developments, there are two or more anchor sections in the annuloplasty ring 800. The anchors 804 are integral to each anchor section. Each anchor section may include two or more anchors 804. Additionally, multiple anchor sections may be placed in parallel with each other to affect a more secure attachment of the ring to the native annulus.

The anchors 804 are superelastic such that applying sufficient stress places the anchors 804 into an introduction configuration and releasing the stress allows the anchors 804 to resume their respective deployed configurations. In certain developments, the anchors 804 are retracted inside the body member 802 of the annuloplasty ring 800 in the introduction configuration during insertion of the annuloplasty ring 800 through the catheter. In such developments, the anchors 804 may be selectively deployed at a desired time (e.g., after the annuloplasty ring 800 is properly positioned against, or in abutment with, the annulus of the heart valve). In certain developments, the superelastic property of the anchors 804 is used to self-propel the anchors 804 into the annulus of the heart valve. The superelastic property of the anchors 804, combined with the curvature of the anchors, causes the anchors 804 to spring into tissue with extra force. The anchors 804 may be configured to be deployed from within the body member 802 through anchor windows 810.

The ring closure lock 812 is used to secure two open ends of the annuloplasty ring 800 to form a closed ring of the operable geometry. In certain developments, the ring closure lock 812 includes a female snap and a male snap. Examples of ring closure locks are provided in U.S. Patent Application No. 13/779,478.

The pivot 814 is used to automatically rotate the annuloplasty ring 800 after it exits the catheter within the heart to align the plane of the annuloplasty ring 800 (in the annular operable geometry) with the plane of the heart valve. The annuloplasty ring 800 is pushed from the catheter in a direction that is substantially perpendicular to the plane of the heart valve (e.g., parallel to the general direction of blood flow through the valve). Upon exiting the catheter, the annuloplasty ring 800 is rotated at or about the pivot 814 to allow proper positioning of the annuloplasty ring 800 against the annulus.

FIG. 8B is a schematic diagram illustrating a cutting pattern 850 used for laser processing a hypotube to form the body member 802 of the annuloplasty ring 800 shown in FIG. 8A. The cutting pattern 850 corresponds to the entire body member 802 as if the body member 802 were cut along a longitudinal axis and unrolled. The cutting pattern 850 enables cutting the hypotube to form the plurality of anchor regions 806 and expansion regions 808. The cutting pattern 850 shown in FIG. 8B defines the configuration of the plurality of regions 806, 808 and how the regions 806, 808 interact with adjacent regions as the body member 802 transitions from the elongate insertion geometry to the annular operable geometry.

The cutting pattern 850 also enables cutting the body member 802 to form anchor windows 810 through which the plurality of anchors 804 are deployed. The cutting pattern 850 may also enable cutting the body member 802 to form one or more through holes 816, 818 at each end to allow one or more pins (not shown) to couple male and/or female components of the ring closure lock 812 to respective ends of the body member 802.

In certain developments, deployment of the anchors is accomplished using internal anchor members that are selectively movable within the body member of an annuloplasty ring. For example, FIGS. 9A, 9B, 9C, 9D, and 9E are simplified views of an internal anchor ribbon 900 and an internal anchor ribbon 902, each anchor ribbon including a plurality of anchors 904a, 904b (referred to collectively as anchors 904). The anchors 904 are integral to each anchor ribbon 900, 902. Each anchor ribbon 900, 902 includes two or more anchors 904. The anchors 904 may be affixed (e.g., laser welded) to the internal anchor ribbons 900, 902 or directly cut into the internal anchor ribbons 900, 902 (as discussed with respect to FIGS. 9A, 9B, and 9C). The anchors 904 may be similar to anchor 604 discussed above in reference to FIG. 8A. For example, the anchors 904 may be superelastic such that applying sufficient stress places the anchors 904 into an introduction configuration and releasing the stress allows the anchors 904 to resume their respective deployed configurations. The anchors 904 may be selectively deployed at a desired time (e.g., after the annuloplasty ring is properly positioned against the annulus of the heart valve). In certain developments, the superelastic property of the anchors 904 is used to self-propel the anchors 904 into the annulus of the heart valve. The superelastic property of the anchors 904, combined with the curvature of the anchors 904, causes the anchors 904 to spring into tissue with extra force.

In some developments, the anchors 904 are in the same plane as the anchor ribbons 900, 902. In other developments, the anchors deploy at an angle to the plane of the anchor ribbons 900, 902, such as 10° or 45°. Additionally, as shown in FIG. 9C, multiple anchor ribbons may be placed in parallel with each other to affect a more secure attachment of the ring to the native annulus.

The internal anchor ribbons 900, 902 may be slid (e.g., using wires or sutures accessible through the catheter) within the body member of the annuloplasty ring. To reduce friction between the internal anchor ribbons 900, 902 and the body member, certain developments include an internal glide ribbon 910. The internal glide ribbon 910 may include a low-friction material (e.g., as a coating or covering) such as PTFE or other polymer.

FIG. 9A is a schematic diagram illustrating a top view of the anchors 904 cut into the internal anchor ribbons 900, 902 shown in the elongate insertion geometry according to one development. In this example, a laser is used to cut the anchors 904 along a first side 912, a second side 914 (e.g., in a pointed or tip shape), and a third side 916, while leaving a fourth side 918 of the anchor 904 uncut and attached to the internal anchor ribbons 900, 902. After cutting, the anchors 904 are heat set to the desired memorized shape for the deployed configuration. For example, FIG. 9B is a schematic diagram illustrating a side view of the internal anchor ribbons 900, 902 in the elongate insertion geometry and the anchors 904 in a deployed configuration according to one development. The amount of curvature in the deployed configuration of the anchors 904 may depend on the particular application. In the example shown in FIG. 9B, the anchors 904 has a "wave" structure. Additionally, internal anchor ribbons 900, 902 are arranged so that the anchors 904a of internal anchor ribbon 900 point in the opposite direction from the anchors 904b of internal anchor ribbon 902. When deployed, the opposing anchors 904a, 904b provide improved anchoring when compared to anchors that all point in the same direction. FIG. 9C is a schematic diagram showing the internal anchor ribbons 900, 902 arranged side by side. When arranged side by side, the anchors 904 overlap, thereby providing improved anchoring. FIG. 9D is a schematic diagram illustrating a top view of the internal glide ribbon 910, and FIG. 9E is a schematic diagram illustrating a side view of the internal glide ribbon 910, in the elongate insertion geometry according to one development.

In one development, the anchor ribbons 900, 902 include a superelastic shape memory material (e.g., Nitinol) that is heat set to the same memorized annular shape as the body member 802 (shown in FIG. 8A as D-shaped). In addition, or in other developments, the internal glide ribbon 910 includes a superelastic shape memory material (e.g., Nitinol) that is heat set to the same memorized annular shape as the body member 802 (shown in FIG. 8A as D-shaped). Thus, certain developments include four D-shaped superelastic members (the body member 802, the internal anchor ribbons 900, 902, and the internal glide ribbon 910), which cooperate to shape and increase the rigidity of the ring 600.

FIGS. 10A, 10B, and 10C are simplified schematics illustrating cross-section side views of an annuloplasty ring 1000 before (FIG. 10A), partway through (FIG. 10B) and after (FIG. 10C) deployment of the anchors 904 shown in FIG. 9C according to one development. Artisans will recognize from the disclosure herein that the anchors 904 are generally deployed when the ring 1000 is in the annular operable geometry. The ring 1000 may be similar to the annuloplasty ring 800 discussed above in reference to FIG. 8A.

The illustrated ring 1000 includes an outer tube 1002 (e.g., the body member 802 shown in FIG. 8A) including a plurality of anchor deployment windows 1010. During the manufacturing of the ring 1000 and before the ring 1000 is loaded into the catheter, the internal anchor ribbons 900, 902 and the internal glide ribbon 910 are inserted into the outer tube 1002 in a position where the anchors 904 are prevented from exiting through the windows 1010. As shown in FIG. 10A, inserting the internal anchor ribbons 900, 902 into the outer tube 1002 prevents the anchors 904 from assuming their fully curved deployed configuration. In the illustrated development, each anchor 904a of anchor ribbon 900 share an anchor deployment window 1010 with an anchor 904b of anchor ribbon 902. In other developments, every anchor 904a, 904b has its own anchor deployment window 1010.

For deploying the anchors 904, the internal anchor ribbons 900, 902 may include (or may be attached to) a hook or loop 1014 for engaging a wire or suture 1016 that may be pulled by a user through the catheter. As the anchors 904a of the internal anchor ribbon 900 point in the opposite direction from the anchors 904b of the internal anchor ribbon 902, the anchor ribbon 900 is pulled in the direction of arrow 1018 while the anchor ribbon 902 is pulled in the opposite direction (e.g., in the direction of arrow 1020). By pulling the anchor ribbons 900, 902 the tips of each anchor 904 move to a corresponding window 1010. In certain developments, the anchors 904 and windows 1010 are arranged such that the tip of each anchor 904 reaches its respective window 1010 at substantially the same time as the other anchors reach their windows. As shown in FIG. 10B, once the tips of the anchors 904a of the anchor ribbon 900 reach the respective windows 1010, the superelasticity of the anchors 904a propels the internal anchor ribbon 900 in the opposite direction (shown by arrow 1020) as the anchors 904a spring out through the windows 1010 (as indicated by arrow 1022) to resume their curved configurations, thereby driving the anchors 904a into surrounding tissue (e.g., the heart valve annulus). As shown in FIG. 10C, once the tips of the anchors 904b of the anchor ribbon 902 reach the respective windows 1010, the superelasticity of the anchors 904b propels the internal anchor ribbon 902 in the opposite direction (shown by arrow 1018) as the anchors 904 spring out the windows 1010 (as indicated by arrow 1024) to resume their curved configurations, thereby driving the anchors 904b into surrounding tissue (e.g., the heart valve annulus). Thus, the superelasticity of the anchors 904 allows the anchors 904 to be self-propelled into the tissue adjacent or proximate to the ring 1000. While the sequence of FIGS. 10A, 10B, and 10C shows the anchor ribbon 900 deploying before the anchor ribbon 902, artisans will recognize that the anchor ribbon 902 may be deployed first, or the anchor ribbons 900, 902 may be deployed simultaneously.

FIG. 10D is a perspective view of a portion of the annuloplasty ring 1000 shown in FIGS. 10A, 10B, and 10C with a pair of deployed anchors 904 according to one development. The outer tube 802 may include the windows 810 (one window shown in FIG. 10D) described above and schematically represented in FIGS. 10A, 10B, and 10C. In certain developments, the deployed anchors 904 form an angle with a plane of the ring 1000 to provide the anchors 904 with improved access to the valve annulus when the ring is positioned against the valve annulus. During anchor deployment, the plane of the ring 1000 is substantially parallel to the plane of the valve annulus.

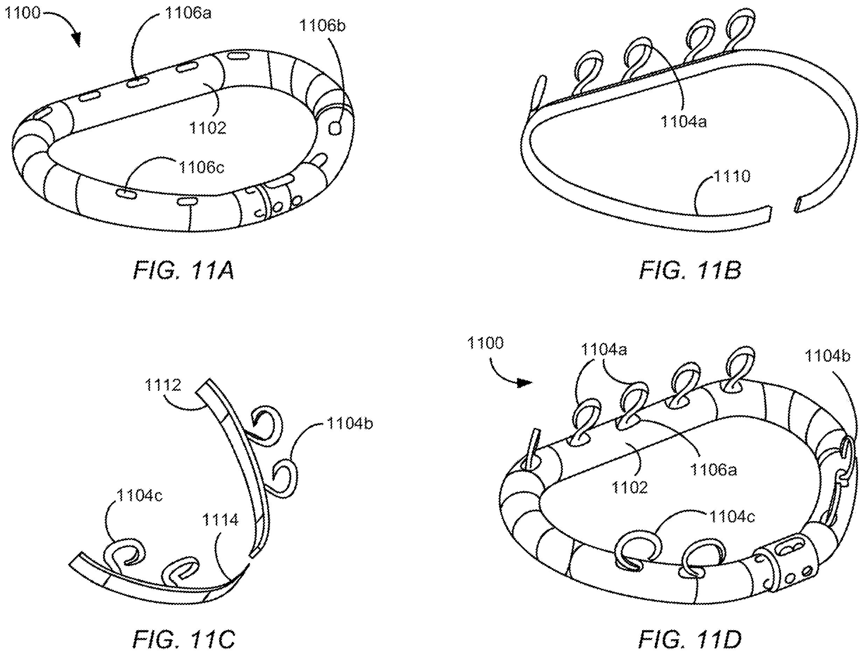

In certain developments, deployment of the anchors is accomplished using a plurality of internal anchor members that are independently deployable within the body member of an annuloplasty ring. For example, FIGS. 11A, 11B, 11C, and 11D are perspective views of an annuloplasty ring 1100 with separately deployable anchor members according to certain developments. The annuloplasty ring 1100 may be similar to annuloplasty rings 800, 1000 discussed above in reference to FIGS. 8A, and 10A, 10B, 10C, and 10D. The annuloplasty ring 1100 includes an outer tube or body member 1102, a plurality of anterior anchors 1104a, a plurality of first posterior anchors 1104b, a plurality of second posterior anchors 1104c, a plurality of anterior anchor windows 1106a, a plurality of first posterior anchor windows 1106b, a plurality of second posterior anchor windows 1106c, an anterior anchor ribbon 1110, a first posterior anchor ribbon 1112, and a second posterior anchor ribbon 1114, each anchor ribbon including a plurality of anchors 1104. The anterior anchors 1104a, first posterior anchors 104b, and second posterior anchors 1104c (collectively referred to as anchors 1104) are integral to their respective anchor ribbons 1110, 1112, 1114. The anchors 1104 may be affixed (e.g., laser welded) to the anchor ribbons 1110, 1112, 1114, respectively, or directly cut into the anchor ribbons 1110, 1112, 1114. Each of the anchor ribbons 1110, 1112, 1114 includes two or more anchors 1104. The anchors 1104 may be similar to anchors 804 discussed above in reference to FIG. 8A. For example, the anchors 1104 may be superelastic such that applying sufficient stress places the anchors 1104 into an introduction configuration and releasing the stress allows the anchors 1104 to resume their respective deployed configurations. The anchors 1104 may be selectively deployed at a desired time (e.g., after the annuloplasty ring 1100 is properly positioned against the annulus of the heart valve). In certain developments, the superelastic property of the anchors 1104 is used to self-propel the anchors 1104 into the annulus of the heart valve. The superelastic property of the anchors 1104, combined with the curvature of the anchors 1104 causes the anchors 1104 to spring into tissue with extra force. The anterior anchor windows 1106a, first posterior anchor windows 1106b, and second posterior anchor windows 1106c (collectively referred to as anchor windows 1106) allow the anchors 1104 to pass through the body member 1102 into the annular tissue.

In some developments, the anchors 1104 are in the same plane as the anchor ribbons 1110, 1112, 1114. In other developments, the anchors deploy at an angle to the plane of the anchor ribbons 1110, 1112, 1114, such as 10° or 45°. Additionally, multiple anchor sections may be placed in parallel with each other to affect a more secure attachment of the ring to the native annulus.

FIG. 11A shows the ring 1100 in the operative configuration with the anchors 1104 in the introduction configuration. FIG. 11D shows the ring 1100 in the operative configuration with the anchors 1104 in the deployed configuration. Artisans will recognize from the disclosure herein that the anchors 1104 are generally deployed when the ring 1100 is in the annular operable geometry and in proximity to the heart valve annulus. The amount of curvature in the deployed configuration of the anchors 1104 may depend on the particular application. In the example shown in FIGS. 11B, 11C, and 11D, the anchors 1104 have a curled structure such that the prong or tip points at the anchor ribbons 1110, 1112, 1114. When deployed, the curled anchors provide secure anchoring in the annular tissue.

FIG. 11B shows the anterior anchor ribbon 1110 with the anterior anchors 1104a arranged in the deployed, operative configuration. FIG. 11C shows the first posterior anchor ribbon 1112 and the second posterior anchor ribbon 1114 with the first posterior anchors 1104b and the second posterior anchors 1104c arranged in the deployed, operative configuration. The anchor ribbons 1110, 1112, 1114 may be slid (e.g., using wires or sutures accessible through the catheter) within the body member 1102 of the annuloplasty ring 1100. To reduce friction between the anchor ribbons 1110, 1112, 1114 and the body member, certain developments include an internal glide ribbon (not shown) that may includes a low- friction material (e.g., as a coating or covering) such as PTFE or other polymer. The anchor ribbons 1110, 1112, 1114 include a superelastic shape memory material (e.g., Nitinol) that is heat set to the same memorized annular shape as the body member 1102 (shown in FIG. 11D as D-shaped). In certain developments, the internal glide ribbon includes a superelastic shape memory material (e.g., Nitinol) that is heat set to the same memorized annular shape as the body member 1102. Thus, certain developments include five D-shaped superelastic members (the body member 1102, the anchor ribbons 110, 1112, 1114, and the internal glide ribbon), which cooperate to increase the rigidity of the ring 1100.

The body member 1102 includes a plurality of anchor deployment windows 1106. During the manufacturing of the ring 1100, and before the ring 1100 is loaded into the catheter, the anchor ribbons 1110, 1112, 1114 are inserted into the body member 1102 in a position where the anchors 1104 are prevented from exiting through the anchor windows 1106. This is referred to as the introduction configuration. As shown in FIG. 11A, the body member 1102 prevents the anchors 1104 from assuming their fully curved deployed configuration.

For deploying the anchors 1104, the anchor ribbons 1110, 1112, 1114 may include (or may be attached to) a hook or loop (not shown) for engaging a wire or suture that may be pulled by a user through the catheter. Pulling the anchor ribbons 1110, 1112, 1114 moves the tips of each anchor 1104 to a deployment window 1106. In certain developments, the anchors 1104 and windows 1106 are arranged such that the tip of each anchor 1104 on an anchor ribbon (e.g., anterior anchor ribbon 1110) reaches its respective window 1106 at substantially the same time as the other anchors 1104 on the anchor ribbon reach their window 1106. Once the tips of the anchors 1104 of an anchor ribbon reach the respective windows 1106, the superelasticity of the anchors 1104 springs them out through the windows 1106 to resume their curved configurations, thereby driving the anchors 1104 into surrounding tissue (e.g., the heart valve annulus). Thus, the superelasticity of the anchors 1104 allows the anchors 1104 to be self-propelled into the tissue adjacent, or proximate, to the ring 1100.

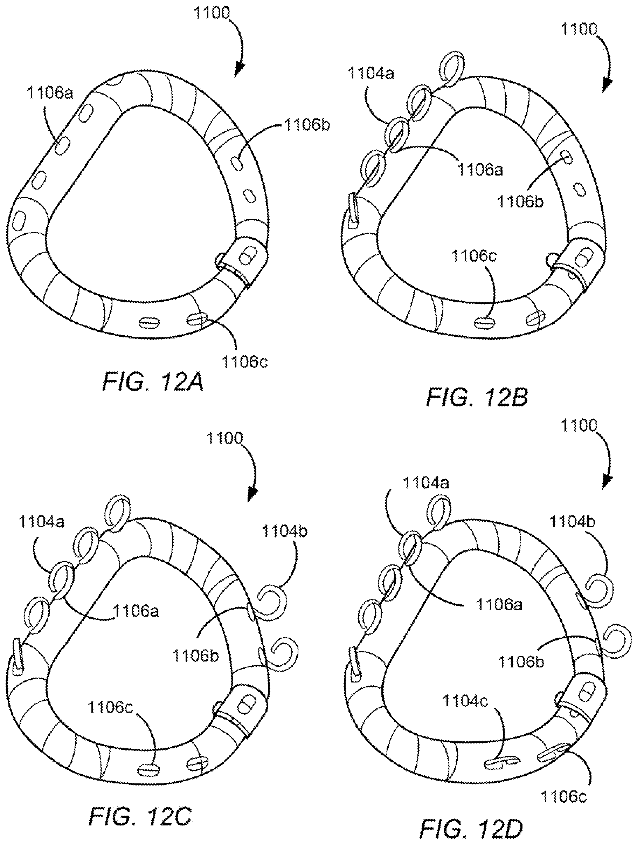

As seen in FIGS. 12A, 12B, 12C, and 12D, the anchor ribbons may be deployed in an independent manner because each anchor ribbon 1110, 1112, or 1114 is independent of the others. Accordingly, the anchors 1104 may be selectively deployed from different locations of the annuloplasty ring 1100 at different times.

FIG. 12A shows the annuloplasty ring 1100 in the operative configuration with the anchors 1104 in the introduction configuration. The annuloplasty ring 1100 is maneuvered into position near the heart valve annulus before the anchors 1104 are deployed.

FIG. 12B shows the annuloplasty ring 1100 with the anterior anchors 1104a deployed. To transition from FIG 12A to FIG. 12B, the anterior anchor ribbon 1110 is pulled within the body member 1102 until the anterior anchors 1104a reach the anterior deployment windows 1106a. Upon reaching the anterior deployment windows 1106a, the anterior anchors 1104a self-propel into the deployment configuration.

FIG. 12C shows the annuloplasty ring 1100 with the anterior anchors 1104a and the first posterior anchors 1104b deployed. To transition from FIG 12B to FIG. 12C, the first posterior anchor ribbon 1112 is pulled within the body member 1102 until the first posterior anchors 1104b reach the first posterior deployment windows 1106b. Upon reaching the first posterior deployment windows 1106b, the first posterior anchors 1104b self-propel into the deployment configuration.

FIG. 12D shows the annuloplasty ring 1100 with the anterior anchors 1104a, the first posterior anchors 1104b and the second posterior anchors 1104c deployed. To transition from FIG 12C to FIG. 12D, the second posterior anchor ribbon 1114 is pulled within the body member 1102 until the second posterior anchors 1104c reach the second posterior deployment windows 1106c. Upon reaching the second posterior deployment windows 1106c, the second posterior anchors 1104c self-propel into the deployment configuration.

Although the sequence of FIGS. 12B, 12C, and 12D shows the anterior anchors 1104a deploying first, then the first posterior anchors 1104b, and finally the second posterior anchors 1140c, artisans will recognize that the anchors 1104a, 1104b, 1104c may be deployed in any order, including simultaneously.

### Example Anchor Shapes and Designs

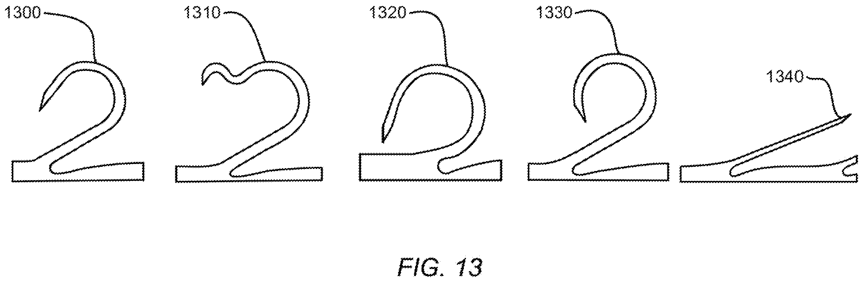

FIG. 13 is a view of various anchor shapes that may be used with the disclosed trans-catheter annuloplasty rings. Anchor shapes 1300, 1320, and 1330 have curved geometries and may be formed from superelastic material, such as Nitinol, so that they self- propel into annulus tissue. The variations in curvature and shape allow for the anchors to deploy into the annular tissue at differing depths. Anchor shape 1310 has a "wavy" geometry. The wave shape gives better grip into the tissue in certain environments. Anchor shape 1340 has a straight geometry and may be propelled into annular tissue through application of mechanical force (e.g., by inflating a balloon behind anchor 1340 or in the center of the annuloplasty ring). Artisans will recognize from the disclosure herein that combinations of barb designs and/or deployed configurations may also be used. In some developments, all of the anchors of an annuloplasty rings are of the same shape. In other developments, an annuloplasty ring may employ a plurality of anchor shapes. For example, one anchor shape may be used to anchor the annuloplasty ring into the anterior portion of the heart valve annulus while a different anchor shape may be used to anchor the annuloplasty ring into the posterior portion of the heart valve annulus. The selected anchor shape or geometry may depend on the particular application.

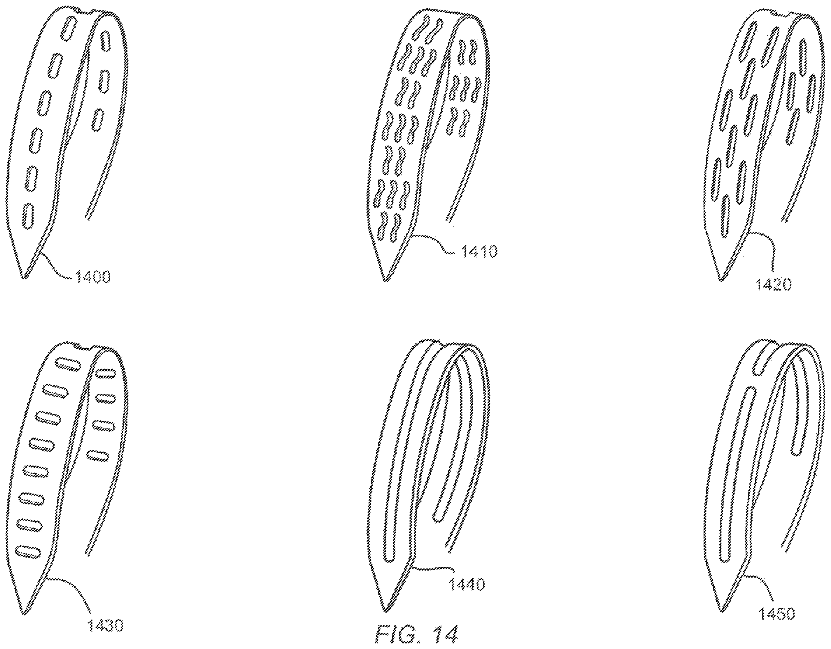

FIG. 14 is a view of various anchor designs that may be used with the disclosed trans-catheter annuloplasty rings. The different designs may be employed to give an anchor greater flexibility or strength, and/or to enhance anchoring within the heart tissue. Controlling the strength of the anchors provides improved tissue penetration by using the strength of the anchors as a driving mechanism. Controlling the elasticity of the anchors allows the anchors to recover from the configuration inside the catheter, when the anchors are in their original cut patter, to their final (deployed) configuration after heat treatment. Anchor design 1400 employs ovoid or rounded-rectangular cutouts along the length of the anchor to provide the anchor with high strength and low elasticity. Anchor design 1410 employs wave cutouts along the length of the anchor to provide the anchor with medium strength and high elasticity (e.g., so as to better act like a spring). Anchor design 1420 employs three offset lines of ovoid or rounded-rectangular cutouts along the length of the anchor to provide the anchor with medium strength and high elasticity (e.g., so as to better act like a spring). Anchor design 1430 employs ovoid or rounded-rectangular cutouts along the length of the anchor. The cutouts of anchor design 1430 are oriented perpendicularly to the length of the anchor (and to the cutouts of anchor design 1400). Anchor design 1440 employs a single ovoid or rounded-rectangular cutout along the length of the anchor to provide medium strength and medium elasticity to the anchor. Anchor design 1450 employs two ovoid or rounded-rectangular cutouts along the length of the anchor. Artisans will recognize from the disclosure herein that combinations of barb designs and/or deployed configurations may also be used. The selected anchor design may depend on the particular application.

### Example Deployment Developments

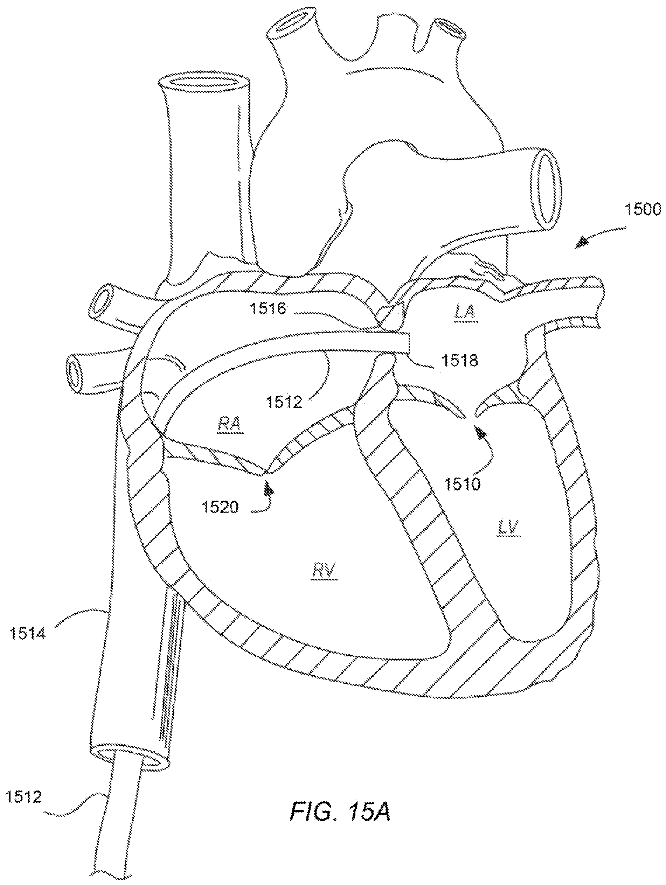

As discussed above, the annuloplasty ring developments disclosed herein are configured for percutaneous transcatheter delivery and fixation to heart valves. The annuloplasty rings may be delivered through a catheter to the mitral valve, for example, using a trans-septal approach, a retrograde approach, or a trans-apical approach. For example, FIG. 15A is a schematic diagram illustrating a trans-septal approach for endovascular delivery of an annuloplasty ring (not shown) to the mitral valve 1510 of a heart 1500 according to one development. For illustrative purposes, a partial cross-section of the heart 1500 is illustrated to show the right atrium RA, right ventricle RV, left atrium LA, and left ventricle LV. For clarity, certain features (e.g., papillary muscles and chordae tendineae) are not shown. In the trans-septal approach shown in FIG. 15A, the left atrium LA is approached by advancement of a catheter 1512 through the inferior vena cava 1514, into the right atrium RA, across the interatrial septum 1516, and into the left atrium LA. The annuloplasty ring may then be delivered through the catheter 1512 into the atrium and anchored to the annulus of the mitral valve 1510.

As shown in FIG. 15A, the catheter 1512 is delivered percutaneously into the heart 1500. A guiding sheath (not shown) may be placed in the vasculature system of the patient and used to guide the catheter 1512 and its distal end 1518 to a desired deployment site. In some developments, a guide wire (not shown) is used to gain access through the superior or inferior vena cava 1514, for example, through groin access for delivery through the inferior vena cava 1514. The guiding sheath may be advanced over the guide wire and into the inferior vena cava 1514 shown in FIG. 15A. The catheter 1512 may be passed through the right atrium RA and toward the interatrial septum 1516. Once the distal end 1518 of the catheter 1512 is positioned proximate to the interatrial septum 1516, a needle or piercing member (not shown) is advanced through the catheter 1512 and used to puncture the fossa ovalis or other portion of the interatrial septum 1516. In some developments, the catheter 1512 is dimensioned and sized to pass through the fossa ovalis without requiring a puncturing device. That is, the catheter 1512 may pass through the natural anatomical structure of the fossa ovalis into the left atrium LA.

Similarly, any chamber (LV, RV, LA, RA) of the heart 1500 may be approached through the inferior vena cava 1514. For example, the right ventricle RV may be approached through the inferior vena cava 1514, into the right atrium RA, and through the tricuspid valve 1520. A variety of other endovascular approaches may also be used.

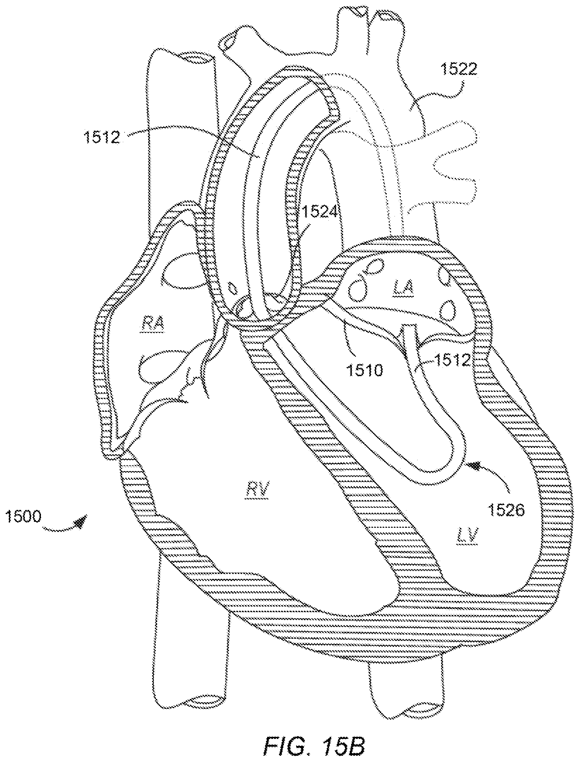

FIG. 15B is a schematic diagram illustrating an example retrograde approach of an annuloplasty ring (not shown) to the mitral valve 1510 of a heart 1500 according to another development. In FIG. 15B, a femoral approach is shown wherein the delivery catheter 1512 is advanced through the aorta 1522 and the aortic valve 1524. Typically, the catheter 1512 is advanced through a sheath positioned within the femoral artery (not shown). Under fluoroscopy or other methods of guidance, the distal end of the catheter 1512 is guided within the left ventricle LV and turned (e.g., as shown with a "U-turn" 1526) within the left ventricle LV so as to pass through the leaflets of the mitral valve 1510 and into the left atrium LA. After verification of the appropriate positioning of the catheter 1512, a guide wire (not shown) may be inserted through the catheter 1512 into the left atrium LA, which may then be used to guide one or more other catheters into the left atrium LA for delivering and anchoring the annuloplasty ring to the annulus of the mitral valve 1510.

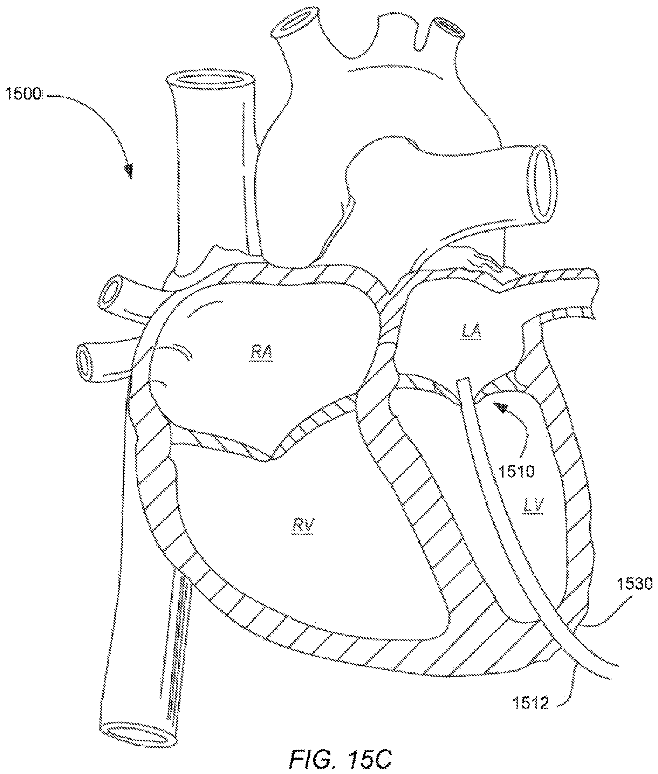

FIG. 15C is a schematic diagram illustrating an example trans-apical approach of an annuloplasty ring (not shown) to the mitral valve 1510 of a heart 1500 according to another development. In this example, the catheter 1512 is shown passing through the apex 1530 of the heart 1500, through the left ventricle LV, through the leaflets of the mitral valve 1510, and into the left atrium. The annuloplasty ring may be delivered through the catheter 1512 into the left atrium LA and anchored to the annulus of the mitral valve 1510. In one development, a needle or trocar may be used to puncture through the apex 1530 to create a small opening through which a guide wire (not shown) can be inserted through the left ventricle LV into the left atrium LA. Then, the guide wire may be used to guide successively larger and stiffer catheters so as to gradually increase the size of the opening in the apex 1530 of the heart 1500.

In some developments, an annuloplasty ring will comprise a pivot used to rotate the annuloplasty ring after it exits the catheter within the heart to align the plane of the annuloplasty ring (in the annular operable geometry) with the plane of the heart valve. The annuloplasty ring is pushed from the catheter in a direction that is substantially perpendicular to the plane of the heart valve (e.g., parallel to the direction of blood flow). Upon exiting the catheter, annuloplasty ring may be rotated via the pivot to allow the annuloplasty ring to be properly positioned against the annulus. In some developments, the annuloplasty ring is expanded at one or more expansion region(s) and pressed against the valve annulus (e.g., using a balloon) before deploying the anchors. The act of deploying the anchors drives the anchors into the tissue. Fluoroscopy, ultrasound, and/or other imaging techniques may be used to assist in proper positioning of the annuloplasty ring against the heart valve annulus.

Prior to deploying the anchors, the annuloplasty ring may be adjusted in the anterior-posterior (A-P) direction to for proper placement. Examples of an annuloplasty ring adjustable in the A-P direction are provided in U.S. Patent Application No. 13/779,478. After the anchors are deployed into the annulus tissue, the annuloplasty ring is allowed to contract thereby reducing the heart valve annulus dimensions and reducing regurgitation through the heart valve.

FIGS. 16A, 16B, 16C, and 16D are schematic diagrams illustrating transcatheter delivery of an annuloplasty ring 1602 from a delivery system 1600 according to certain developments. FIG. 16A illustrates a perspective view of a distal end 1610 of the delivery system 1600. FIG. 16A is a perspective view of the annuloplasty ring 1602 in the elongate insertion geometry and partially deployed from the distal end 1610 of a delivery catheter 1614 in a first deployment stage. In the first stage, the annuloplasty ring 1602 may be still substantially in the elongate insertion geometry. As shown in FIG. 16A, a first suture 1619 for snapping together the ends of the annuloplasty ring 1602 passes through a male snap 1612 of a ring closure lock 1650 (shown in FIG. 16C).

FIG. 16B is a perspective view of the annuloplasty ring 1602 in a second stage of partial deployment from the delivery catheter 1614. In the second stage, the portion of the annuloplasty ring 1602 that has exited the delivery catheter 1614 has begun to transition (due to the shape memory materials used in the annuloplasty ring 1602) from the elongate insertion geometry to the annular operable geometry.

FIG. 16C is a perspective view of the annuloplasty ring 1602 in a third stage of deployment in which a ring shuttle 1616 of the delivery system 1600 has substantially pushed the annuloplasty ring 1602 out of the delivery catheter 1614, but the plane of the annuloplasty ring 1602 is still aligned with (e.g., approximately parallel to) the longitudinal axis of the delivery catheter 1614. In FIG. 16C, the annuloplasty ring 1602 may be in a configuration, for example, immediately before a ring deployment wire 1623 cooperates with the pivot 1608 to rotate the annuloplasty ring 1602 (see FIG. 16D). In the configuration shown in FIG. 16C, the distal end of the ring deployment wire 1623 includes a bend or hook 1632 as it passes through a hole in the pivot 1608. The ring deployment wire 1623 includes a superelastic shape memory material (e.g., Nitinol), and bending the distal end of the ring deployment wire 1623 into the hook 1632 shape spring loads the annuloplasty ring 1602 within the outer jacket delivery catheter 1614 such that the annuloplasty ring 1602 automatically rotates about the pivot 1608 upon exiting the outer jacket delivery catheter 1614. At this third stage of deployment, the hook 1632 shape formed in the superelastic ring deployment wire 1623 is ready to unload (return to a heat-set memorized straight configuration) as soon as the delivery catheter 1614 no longer prevents it from doing so. The suture 1619 may be utilized to draw together the male components 1652 and female components 1654 of a ring closure lock 1650.

FIG. 16D is a perspective view of the annuloplasty ring 1602 in a fourth stage of deployment in which the plane of the annuloplasty ring 1602 (in its annular operable geometry) has been changed to be perpendicular to the longitudinal axis of the delivery catheter 1614. As shown in FIG. 16D, the superelastic ring deployment wire 1623 has returned to its heat set (memorized) straight configuration. At this fourth stage of deployment, the plane of the annuloplasty ring 1602 is configured to be parallel to the plane of the heart valve annulus. In situ within the heart, a longitudinal axis of the delivery catheter 1614 is oriented parallel to the direction of blood through the valve and approximately perpendicular to the plane of the heart valve. The annuloplasty ring 1602, when oriented such that the plane of the annuloplasty ring 1602 is transverse to (and perpendicular or approximately perpendicular to) the longitudinal axis of the delivery catheter 1614, is oriented such that the plane of the annuloplasty ring 1602 is parallel or approximately parallel to the plane of the heart valve.

In further stages of deployment, the annuloplasty ring 1602 may be expanded and/or pressed against the heart valve annulus before deploying the anchors (such as the curved anchors 104 shown in FIGS. 1A and 1B). As discussed above, certain anchor developments propel themselves into the tissue of the heart valve annulus upon being deployed. In other developments, the anchors (such as the linear anchors 1340 shown in FIG. 13) may be deployed before pressing the annuloplasty ring 1602 against the annulus. After the annuloplasty ring 1602 is anchored to the heart valve annulus and transitioned to the contracted state, the ring deployment wire 1623 may be pulled from the hole in the pivot 1608 to release the annuloplasty ring 1602 from the ring shuttle 1616. Any remaining sutures, such as the first suture 1619, may also be cut and/or pulled from the annuloplasty ring 1602 before the delivery catheter 1614 is removed from the heart.

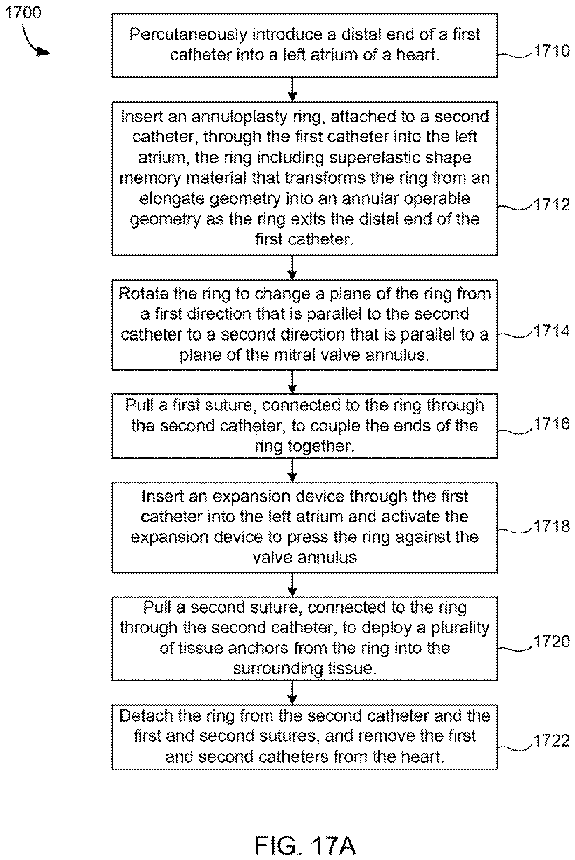

FIG. 17A is a flowchart of a method 1700 for repairing a defective heart valve according to one development. The method 1700 includes percutaneously introducing 1710 a distal end of a first catheter into a left atrium of a heart and inserting 1712 an annuloplasty ring, attached to a second catheter, through the first catheter into the left atrium. The ring includes a superelastic shape memory material that transforms the ring from an elongate insertion geometry to an annular operable geometry as the ring exits the distal end of the first catheter. The method 1700 further includes rotating 1714 the ring to change a plane of the ring from a first direction that is parallel to the second catheter to a second direction that is parallel to a plane of the mitral valve annulus, and pulling 1716 a first suture, connected to the ring through the second catheter, to couple the ends of the ring together. The method 1700 includes inserting 1718 an expansion device through the first catheter into the left atrium and activating the expansion device to press the ring against the valve annulus. Then, pulling 1720 a second suture, connected to the ring through the second catheter, to deploy a plurality of tissue anchors from the ring into the surrounding tissue. In some developments, the step 1720 of deploying anchors comprises retracting a retaining ribbon from across the anchors thereby causing the anchors to deploy as described above in relation to FIGS. 4A, 4B, 4C, 4D, and 4F. In other developments, the step 1720 of deploying anchors comprises rotating an inner tube within an outer cover thereby causing the anchors to deploy through windows in the outer cover as described above in relation to FIGS. 5A, 5B, 5C, and 5D. The method 1700 further includes detaching 1722 the ring from the second catheter and the first and second sutures, and remove the first and second catheters from the heart.

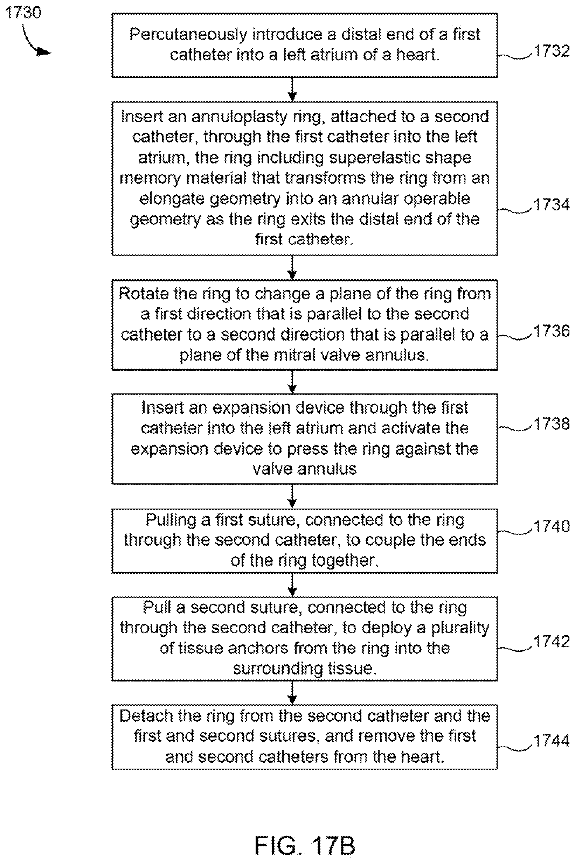

FIG. 17B is a flowchart of a method 1730 for repairing a defective heart valve according to another development. The method 1730 includes percutaneously introducing 1732 a distal end of a first catheter into a left atrium of a heart, and inserting 1734 a segmented annuloplasty ring, attached to a second catheter, through the first catheter into the left atrium. The ring includes superelastic shape memory material that transforms the ring from an elongate insertion geometry to an annular operable geometry as the ring exits the distal end of the first catheter. The method 1730 further includes automatically rotating 1736 the ring to change a plane of the ring from a first direction that is parallel to the second catheter to a second direction that is parallel to a plane of the mitral valve annulus, and inserting 1738 an expansion device through the first catheter into the left atrium and activating the expansion device to press the ring against the valve annulus. Pressing the ring against the annulus at this stage allows the subsequent deployment of the anchors to propel the anchors into the annulus tissue. Thus, the method 1730 further includes pulling 1740 a first suture, connected to the ring through the second catheter, to deploy a plurality of tissue anchors from the ring. Each of the plurality of anchors includes a superelastic shape memory material that propels the superelastic anchors into the tissue of the valve annulus. In some developments, the step 1740 of deploying anchors comprises retracting a retaining ribbon from across the anchors thereby causing the anchors to deploy as described above in relation to FIGS. 4A, 4B, 4C, 4D, and 4F. In other developments, the step 1740 of deploying anchors comprises rotating an inner tube within an outer cover thereby causing the anchors to deploy through windows in the outer cover as described above in relation to FIGS. 5A, 5B, 5C, and 5D. The method 1730 further includes pulling 1742 a second suture, connected to the ring through the second catheter, to couple the ends of the ring together and cinch the valve annulus to a desired size. The method 1730 also includes detaching 1744 the ring from the second catheter and the first and second sutures, and removing the first and second catheters from the heart.

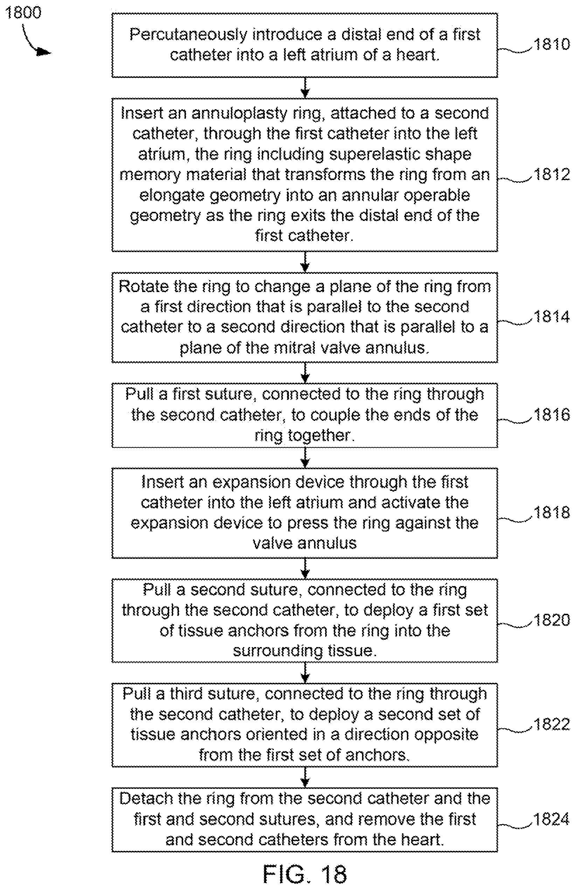

FIG. 18 is a flowchart of a method 1800 for repairing a defective heart valve according to one development. The method 1800 includes percutaneously introducing 1810 a distal end of a first catheter into a left atrium of a heart and inserting 1812 an annuloplasty ring, attached to a second catheter, through the first catheter into the left atrium. The ring includes a superelastic shape memory material that transforms the ring from an elongate insertion geometry to an annular operable geometry as the ring exits the distal end of the first catheter. The method 1800 further includes rotating 1814 the ring to change a plane of the ring from a first direction that is parallel to the second catheter to a second direction that is parallel to a plane of the mitral valve annulus, and pulling 1816 a first suture, connected to the ring through the second catheter, to couple the ends of the ring together. The method 1800 includes inserting 1818 an expansion device through the first catheter into the left atrium and activating the expansion device to press the ring against the valve annulus. Next, pulling 1820 a second suture, connected to the ring through the second catheter, to deploy a first set of tissue anchors from the ring into the surrounding tissue. The method includes pulling 1822 a third suture, also connected to the ring through the second catheter, to deploy a second set of tissue anchors oriented in a direction opposite (anti-parallel) from the first set of anchors into the surrounding tissue. The method 1800 may also include pulling additional sutures, also connected to the ring through the second catheter, to deploy additional sets of tissue anchors, located on additional anchor regions of the annuloplasty ring, into the surrounding tissue. In some developments, three sets of anchors are deployed; each set of anchors attached to an internal anchor ribbon and deployed using a separate suture. In addition, or in other developments, the method 1800 may also include pulling another suture, connected to the ring through the second catheter, to couple the ends of the ring together. The method 1800 further includes detaching 1824 the ring from the second catheter and the first, second, and third sutures, and remove the first and second catheters from the heart.

## Claims

1. An annuloplasty device for transcatheter heart valve repair, the annuloplasty device comprising:
a tubular body member (402) comprising shape memory material, the tubular body member (402) including a plurality of anchors (404);
a retaining ribbon (408) to hold the plurality of anchors (404) in the introduction configuration and to selectively release the plurality of anchors (404) to the deployed configuration; and
**characterized in that**
the tubular body member (402) includes a plurality of anchors (404) cut therein, each of the plurality of anchors (404) cut along a circumference of the tubular body member (402) such that, in an introduction configuration, the anchors (404) are in a radial plane of the tubular body member (402) and are concentric with the tubular body member (402), the plurality of anchors (404) heat treated to a deployed configuration that extends away from the radial plane of the tubular body member (402); and
the annuloplasty device further comprises one or more retainer features (406) extending from an outer surface of the tubular body member (402), the one or more retainer features (406) to hold the retaining ribbon (408) against the outer surface of tubular body member (402) and the plurality of anchors (404) in introduction configuration, and the one or more retainer features (406) allowing the retaining ribbon (408) to slide along the surface of the tubular body member (402) to selectively release one or more of the plurality of anchors (404).

2. The annuloplasty device of claim 1, wherein the plurality anchors (404) in the deployed configuration are curved in an opposite direction from that of the radial plane of the tubular body member (402) such that a transition from the introduction configuration to the deployed configuration increases a force of a superelastic property of the shape memory material to drive the plurality of anchors (404) into tissue of a heart valve annulus.

3. The annuloplasty device of claim 1, wherein the tubular body member (402) is configured to change from an elongate insertion geometry to an annular operable geometry, the annuloplasty device configured to pass through a catheter and into a heart chamber in the elongate insertion geometry and to be anchored to a heart valve annulus in the annular operable geometry.

4. The annuloplasty device of claim 1, wherein the retaining ribbon (408) is configured to prevent the plurality of anchors (404) from deploying until the retaining ribbon (408) has been retracted.

5. The annuloplasty device of claim 1, wherein when the retaining ribbon (408) is positioned between the plurality of anchors (404) and the retainer features (406) it thereby holds the plurality of anchors (404) flush with the tubular body member (402).

6. The annuloplasty device of claim 1, wherein the retaining ribbon (408) is configured to restrain the plurality of anchors (404) from being deployed in a first position where the one or more retainer features are holding the retaining ribbon against the outer surface of tubular body member and the plurality of anchors in introduction configuration, and to allow the plurality of anchors (404) to self-deploy into an annulus tissue in a second position where the retaining ribbon has been retracted past the plurality of anchors.

7. The annuloplasty device of claim 1, wherein the plurality of anchors (404) are configured to deploy via retracting the retaining ribbon prior to the tubular body member (402) being placed in an operable geometry of a heart valve annulus.

## Patentansprüche

1. Anuloplastie-Vorrichtung für Transkatheter-Herzklappen-Reparatur, wobei die Anuloplastie-Vorrichtung Folgendes umfasst:
ein Rohrkörperelement (402), das ein Formgedächtnismaterial umfasst, wobei das Rohrkörperelement (402) eine Vielzahl von Verankerungen (404) einschließt;
ein Halteband (408), um die Vielzahl von Verankerungen (404) in der Einführkonfiguration zu halten und um die Vielzahl von Verankerungen (404) für die Einsatzkonfiguration selektiv freizulassen; und
**dadurch gekennzeichnet, dass**
das Rohrkörperelement (402) eine Vielzahl von Verankerungen (404) einschließt, die dahinein eingeschnitten sind, wobei jede der Vielzahl von Verankerungen (404) entlang einem Umfang des Rohrkörperelements (402) derart eingeschnitten ist, dass in einer Einführkonfiguration die Verankerungen (404) in einer radialen Ebene des Rohrkörperelements (402) vorliegen und konzentrisch mit dem Rohrkörperelement (402) sind, die Vielzahl von Verankerungen (404) zu einer Einsatzkonfiguration wärmebehandelt wird, die sich von der radialen Ebene des Rohrkörperelements (402) wegstreckt; und
die Anuloplastie-Vorrichtung weiter eine oder mehrere Haltefunktionen (406) umfasst, die sich von einer äußeren Oberfläche des Rohrkörperelements (402) erstrecken, wobei die eine oder mehreren Haltefunktionen (406) das Halteband (408) gegen die äußere Oberfläche des Rohrkörperelements (402) und die Vielzahl von Verankerungen (404) in Einführkonfiguration halten und die eine oder mehreren Haltefunktionen (406) ermöglichen, dass sich das Halteband (408) entlang der Oberfläche des Rohrkörperelements (402) verschiebt, um eine oder mehrere der Vielzahl von Verankerungen (404) selektiv freizulassen.

2. Anuloplastie-Vorrichtung nach Anspruch 1, wobei die Vielzahl von Verankerungen (404) in der Einsatzkonfiguration in einer entgegengesetzten Richtung von der der radialen Ebene des Rohrkörperelements (402) derart gebogen ist, dass ein Übergang von der Einführkonfiguration zu der Einsatzkonfiguration eine Kraft einer superelastischen Eigenschaft des Formgedächtnismaterials erhöht, um die Vielzahl von Verankerungen (404) in Gewebe eines Herzklappenanulus zu treiben.

3. Anuloplastie-Vorrichtung nach Anspruch 1, wobei das Rohrkörperelement (402) zur Änderung von einer länglichen Einfügegeometrie zu einer ringförmigen funktionsfähigen Geometrie konfiguriert ist, wobei die Anuloplastie-Vorrichtung zum Durchgang durch einen Katheter und in eine Herzkammer in der länglichen Einfügegeometrie und zur Verankerung an einem Herzklappenanulus in der ringförmigen funktionsfähigen Geometrie konfiguriert ist.

4. Anuloplastie-Vorrichtung nach Anspruch 1, wobei das Halteband (408) konfiguriert ist zu verhindern, dass die Vielzahl von Verankerungen (404) eingesetzt werden, bevor das Halteband (408) zurückgezogen wurde.

5. Anuloplastie-Vorrichtung nach Anspruch 1, wobei das Halteband (408), wenn es zwischen die Vielzahl von Verankerungen (404) und die Haltefunktionen (406) positioniert ist, dadurch die Vielzahl von Verankerungen (404) bündig mit dem Rohrkörperelement (402) hält.

6. Anuloplastie-Vorrichtung nach Anspruch 1, wobei das Halteband (408) konfiguriert ist, um die Vielzahl von Verankerungen (404) zurückzuhalten, dass sie in einer ersten Position eingesetzt wird, wo die eine oder mehreren Haltefunktionen das Halteband gegen die äußere Oberfläche des Rohrkörperelements und die Vielzahl von Verankerungen in Einführkonfiguration halten, und um zu ermöglichen, dass sich die Vielzahl von Verankerungen (404) in ein Anulusgewebe in einer zweiten Position, wo das Halteband vorbei an der Vielzahl von Verankerungen zurückgezogen wurde, selbst einsetzt.

7. Anuloplastie-Vorrichtung nach Anspruch 1, wobei die Vielzahl von Verankerungen (404) konfiguriert ist, um über das Zurückziehen des Haltebands einzusetzen, bevor das Rohrkörperelement (402) in einer funktionsfähigen Geometrie eines Herzklappenanulus positioniert wird.

## Revendications

1. Dispositif d'annuloplastie pour la réparation d'une valvule cardiaque transcathéter, le dispositif d'annuloplastie comprenant :
un organe formant corps tubulaire (402) comprenant un matériau à mémoire de forme, l'organe formant corps tubulaire (402) incluant une pluralité d'ancres (404) ;
un ruban de retenue (408) pour maintenir la pluralité d'ancres (404) dans la configuration d'introduction et pour libérer sélectivement la pluralité d'ancres (404) vers la configuration déployée ; et
**caractérisé en ce que**
l'organe formant corps tubulaire (402) inclut une pluralité d'ancres (404) découpées dans celui-ci, chaque ancre de la pluralité d'ancres (404) découpée le long d'une circonférence de l'organe formant corps tubulaire (402) de sorte que, dans une configuration d'introduction, les ancres (404) sont dans un plan radial de l'organe formant corps tubulaire (402) et sont concentriques avec l'organe formant corps tubulaire (402), la pluralité d'ancres (404) traitées à la chaleur vers une configuration déployée qui s'étend en s'éloignant du plan radial de l'organe formant corps tubulaire (402) ; et
le dispositif d'annuloplastie comprend en outre un ou plusieurs éléments de retenue (406) s'étendant d'une surface externe de l'organe formant corps tubulaire (402), les un ou plusieurs éléments de retenue (406) pour maintenir le ruban de retenue (408) contre la surface externe de l'organe formant corps tubulaire (402) et la pluralité d'ancres (404) dans la configuration d'introduction, et les un ou plusieurs éléments de retenue (406) permettant au ruban de retenue (408) de glisser le long de la surface de l'organe formant corps tubulaire (402) pour libérer sélectivement une ou plusieurs ancres de la pluralité d'ancres (404).

2. Dispositif d'annuloplastie selon la revendication 1, dans lequel la pluralité d'ancres (404) dans la configuration déployée sont courbées dans une direction opposée à celle du plan radial de l'organe formant corps tubulaire (402) de sorte qu'une transition de la configuration d'introduction vers la configuration déployée augmente une force d'une propriété superélastique du matériau à mémoire de forme pour diriger la pluralité d'ancres (404) jusque dans le tissu d'un anneau de valvule cardiaque.

3. Dispositif d'annuloplastie selon la revendication 1, dans lequel l'organe formant corps tubulaire (402) est configuré pour changer d'une géométrie d'insertion allongée à une géométrie de fonctionnement annulaire, le dispositif d'annuloplastie configuré pour passer à travers un cathéter et jusque dans une chambre cardiaque dans la géométrie d'insertion allongée et pour être ancré dans un anneau de valvule cardiaque dans la géométrie de fonctionnement annulaire.

4. Dispositif d'annuloplastie selon la revendication 1, dans lequel le ruban de retenue (408) est configuré pour empêcher la pluralité d'ancres (404) de se déployer jusqu'à ce que le ruban de retenue (408) ait été rétracté.

5. Dispositif d'annuloplastie selon la revendication 1, dans lequel quand le ruban de retenue (408) est positionné entre la pluralité d'ancres (404) et les éléments de retenue (406), il retient ainsi la pluralité d'ancres (404) au même niveau que l'organe formant corps tubulaire (402).

6. Dispositif d'annuloplastie selon la revendication 1, dans lequel le ruban de retenue (408) est configuré pour empêcher la pluralité d'ancres (404) d'être déployées dans une première position où les un ou plusieurs éléments de retenue maintiennent le ruban de retenue contre la surface externe de l'organe formant corps tubulaire et la pluralité d'ancres dans la configuration d'introduction, et pour permettre à la pluralité d'ancres (404) de s'auto-déployer jusque dans un tissu d'anneau dans une deuxième position où le ruban de retenue a été rétracté devant la pluralité d'ancres.

7. Dispositif d'annuloplastie selon la revendication 1, dans lequel la pluralité d'ancres (404) sont configurées pour se déployer par rétraction du ruban de retenue avant que l'organe formant corps tubulaire (402) soit placé dans une géométrie de fonctionnement d'un anneau de valvule cardiaque.
